# EUROPEAN PATENT APPLICATION

(11) **EP 3 255 150 A1**
(43) Date of publication of application: **13.12.2017**
(21) Application number: 15881324.6
(22) Date of filing: 28.12.2015
(51) Int. Cl.: C12N 15/70, C12N 9/10, C12P 7/46

(54) **MICROORGANISM CAPABLE OF PRODUCING QUINOLINIC ACID, AND METHOD FOR PRODUCING QUINOLINIC ACID UTILIZING THE MICROORGANISM**

(30) Priority: 03.02.2015 KR 20150016971
(71) Applicant: CJ Cheiljedang Corporation, Seoul 04560 (KR)
(72) Inventor: LEE, Jaemin, Uijeongbu-si Gyeonggi-do 11601 (KR); KIM, Ju Eun, Seoul 07524 (KR); LEE, Jae Hee, Seoul 07516 (KR); CHANG, Jin Sook, Suwon-si Gyeonggi-do 16532 (KR); KIM, So Young, Gwacheon-si Gyeonggi-do 13803 (KR); SHIN, Yong Uk, Yongin-si Gyeonggi-do 16898 (KR)
(74) Representative: Nony
(86) International application number: PCT/KR2015/014344
(87) International publication number: WO 2016/126006

(57) **Abstract**

The present disclosure relates to a microorganism having producing ability of quinolinic acid and a method for producing quinolinic acid using the microorganism.

<110>     CJ CheilJedang Corporation

<120>     A microorganism having an ability to produce quinolinic acid and
          a method for producing a quinolinic acid using the same

<130>     OPA15315-PCT

<150>     KR 10-2015-0016971
<151>     2015-02-03

<160>     43

<170>     KopatentIn 2.0

<210>     1
<211>     347
<212>     PRT
<213>     Klebsiella pneumonia

<400>     1
Met Ser Val Met Phe Asp Pro Glu Thr Ala Ile Tyr Pro Phe Pro Ala
  1               5                  10                  15

Lys Pro Gln Pro Leu Thr Val Asp Glu Lys Gln Phe Tyr Arg Glu Lys
             20                  25                  30

Ile Lys Arg Leu Leu Arg Glu Arg Asp Ala Val Met Val Ala His Tyr
         35                  40                  45

Tyr Thr Asp Pro Glu Ile Gln Gln Leu Ala Glu Glu Thr Gly Gly Cys
     50                  55                  60

Ile Ala Asp Ser Leu Glu Met Ala Arg Phe Gly Ala Arg His Ser Ala
 65                  70                  75                  80

Ser Thr Leu Leu Val Ala Gly Val Arg Phe Met Gly Glu Thr Ala Lys
                 85                  90                  95

Ile Leu Ser Pro Glu Lys Thr Ile Leu Met Pro Thr Leu Asn Ala Glu
            100                 105                 110

Cys Ser Leu Asp Leu Gly Cys Pro Ile Glu Glu Phe Asn Ala Phe Cys
        115                 120                 125

Asp Ala His Pro Asp Arg Thr Val Val Val Tyr Ala Asn Thr Ser Ala
    130                 135                 140

Ala Val Lys Ala Arg Ala Asp Trp Val Val Thr Ser Ser Ile Ala Val
145                 150                 155                 160

Glu Leu Ile Asp His Leu Asp Ser Leu Gly Gln Lys Ile Leu Trp Ala
                165                 170                 175

Pro Asp Arg His Leu Gly Arg Tyr Val Gln Arg Gln Thr Gly Ala Asp
            180                 185                 190

Val Leu Cys Trp Gln Gly Ala Cys Ile Val His Asp Glu Phe Lys Thr
        195                 200                 205

Gln Ala Leu Met Arg Met Lys Ala Leu His Pro Glu Ala Ala Val Leu
    210                 215                 220

Val His Pro Glu Ser Pro Gln Ala Ile Val Glu Met Ala Asp Ala Val

225                 230                 235                 240

Gly Ser Thr Ser Gln Leu Ile Ala Ala Ala Lys Ser Leu Pro Gln Arg
                245                 250                 255

Gln Leu Ile Val Ala Thr Asp Arg Gly Ile Phe Tyr Lys Met Gln Gln
            260                 265                 270

Ala Val Pro Glu Lys Thr Leu Leu Glu Ala Pro Thr Ala Gly Glu Gly
        275                 280                 285

Ala Thr Cys Arg Ser Cys Ala His Cys Pro Trp Met Ala Met Asn Gly
    290                 295                 300

Leu Lys Ala Ile Ala Glu Gly Leu Glu Gln Gly Gly Ala Glu His Glu
305                 310                 315                 320

Ile His Val Asp Glu Ala Leu Arg Thr Gly Ala Leu Ile Pro Leu Asn
                325                 330                 335

Arg Met Leu Asp Phe Ala Ala Thr Leu Arg Gly
            340                 345

<210>    2
<211>    1044
<212>    DNA
<213>    Klebsiella pneumonia

<400>    2
atgagcgtaa tgtttgatcc tgaaacggcg atttatcctt tccctgctaa accgcagccg      60

ctgaccgtcg acgaaaagca gttttaccgc gaaaaaatca agcgcctgct gcgcgagcgc     120

gatgccgtga tggtggcgca ttactacacc gatcctgaaa ttcaacagct ggcggaagag     180

accggcggct gtatcgccga ctcgctggag atggcgcgct ttggcgcccg ccattcggcc     240

tccacgctgc tggtcgccgg ggtgcgtttt atgggggaaa ccgccaaaat tctcagcccg     300

gaaaagacca ttttgatgcc gaccctgaac gccgagtgtt cattggatct gggctgtccg     360

attgaggaat tcaacgcctt ttgcgacgcc catcctgacc gcaccgtcgt ggtctatgcc     420

aatacgtccg ccgcggtgaa ggcccgcgcc gactgggtgg tgacctccag catcgccgtg     480

gaactcattg accatctgga tagtcttggt caaaagatcc tctgggcgcc ggaccgccac     540

cttgggcgtt acgttcagcg tcagaccggc gcagacgtgc tgtgctggca gggggcgtgc     600

atcgtgcacg acgagtttaa aacccaggcg ttgatgcgga tgaaggctct gcatcccgaa     660

gccgccgtgc tggtccatcc cgagtcgccg caggcgatcg ttgagatggc cgatgccgta     720

ggctccacca gccagctgat tgcggcggcg aaaagcctgc cccagcgcca gctgatcgtg     780

gccaccgatc gcggtatttt ctataaaatg cagcaggcgg tgccggagaa aacgctgctg     840

gaagcgccca ccgccggcga aggggcgacc tgccgcagct gcgcgcattg tccgtggatg     900

gcgatgaatg gcctgaaagc cattgccgag gggctcgagc agggcggcgc tgaacatgaa     960

atccatgtcg acgaagcgct gcgaaccggc gcattaattc cccttaaccg gatgctggat    1020

tttgcggcta cactacgggg ataa                                                1044

<210>    3
<211>    297
<212>    PRT
<213>    Escherichia coli

<400>    3
Met Pro Pro Arg Arg Tyr Asn Pro Asp Thr Arg Arg Asp Glu Leu Leu
1               5                   10                  15

Glu Arg Ile Asn Leu Asp Ile Pro Gly Ala Val Ala Gln Ala Leu Arg
            20                  25                  30

Glu Asp Leu Gly Gly Thr Val Asp Ala Asn Asn Asp Ile Thr Ala Lys
        35                  40                  45

Leu Leu Pro Glu Asn Ser Arg Ser His Ala Thr Val Ile Thr Arg Glu
    50                  55                  60

Asn Gly Val Phe Cys Gly Lys Arg Trp Val Glu Glu Val Phe Ile Gln
65                  70                  75                  80

Leu Ala Gly Asp Asp Val Thr Ile Ile Trp His Val Asp Asp Gly Asp
                85                  90                  95

Val Ile Asn Ala Asn Gln Ser Leu Phe Glu Leu Glu Gly Pro Ser Arg
            100                 105                 110

Val Leu Leu Thr Gly Glu Arg Thr Ala Leu Asn Phe Val Gln Thr Leu
        115                 120                 125

Ser Gly Val Ala Ser Lys Val Arg His Tyr Val Glu Leu Leu Glu Gly
    130                 135                 140

Thr Asn Thr Gln Leu Leu Asp Thr Arg Lys Thr Leu Pro Gly Leu Arg
145                 150                 155                 160

Ser Ala Leu Lys Tyr Ala Val Leu Cys Gly Gly Gly Ala Asn His Arg
                165                 170                 175

Leu Gly Leu Ser Asp Ala Phe Leu Ile Lys Glu Asn His Ile Ile Ala
            180                 185                 190

Ser Gly Ser Val Arg Gln Ala Val Glu Lys Ala Ser Trp Leu His Pro
        195                 200                 205

Asp Ala Pro Val Glu Val Glu Val Glu Asn Leu Glu Glu Leu Asp Glu
    210                 215                 220

Ala Leu Lys Ala Gly Ala Asp Ile Ile Met Leu Asp Asn Phe Glu Thr
225                 230                 235                 240

Glu Gln Met Arg Glu Ala Val Lys Arg Thr Asn Gly Lys Ala Leu Leu
                245                 250                 255

Glu Val Ser Gly Asn Val Thr Asp Lys Thr Leu Arg Glu Phe Ala Glu
            260                 265                 270

Thr Gly Val Asp Phe Ile Ser Val Gly Ala Leu Thr Lys His Val Gln
        275                 280                 285

Ala Leu Asp Leu Ser Met Arg Phe Arg
    290                 295

<210>    4
<211>    894
<212>    DNA
<213>    Escherichia coli

<400>    4
atgccgcctc gccgctataa ccctgacacc cgacgtgacg agctgctgga acgcattaat        60

ctcgatatcc ccggcgcggt ggcccaggcg ctgcgggaag atttaggcgg aacagtcgat       120

gccaacaatg atattacggc aaaactttta ccggaaaatt ctcgctctca tgccacggtg       180

atcacccgcg agaatggcgt cttttgcggc aaacgctggg ttgaagaggt gtttattcaa       240

ctggcaggcg acgatgtcac cataatctgg catgtggatg acggcgatgt catcaatgcc       300

aatcaatcct tgttcgaact tgaaggccca tcccgcgtgc tgttaacggg cgaacgcact       360

gcgcttaatt ttgtgcaaac cctttcagga gttgccagta aggtacgcca ctatgtcgaa       420

ttgctggaag gcaccaacac gcagttgttg gatacgcgca aaaccttacc cggcctgcgt       480

tcagctctga aatacgcggt actttgcggc ggcggagcga atcaccgtct ggggctttct       540

gatgccttcc tgatcaaaga aaaccatatt attgcctccg gctcagtgcg ccaggcggtc       600

gaaaaagcgt cctggctgca cccggatgcg ccagtagaag tcgaagtaga gaatctggaa       660

gaacttgatg aagccctgaa agcaggagcc gatatcatca tgctggataa cttcgaaaca       720

gaacagatgc gcgaagccgt caaacgcacc aacggcaagg cgctactgga agtgtctggc       780

aacgtcactg acaaaacact gcgtgaattt gccgaaacgg gcgtggactt tatctccgtc       840

ggtgcgctaa ctaaacacgt acaagcactc gacctttcaa tgcgttttcg ctaa             894

<210>    5
<211>    42
<212>    DNA
<213>    Artificial Sequence

<220>
<223>    Primer

<400>    5
cattatacga acggtacccc cagttgaata aacacctctt ca                           42

<210>    6
<211>    18
<212>    DNA
<213>    Artificial Sequence

<220>
<223>    Primer

<400>    6
tggcggcagg ctaatatt                                                    18


<210>    7
<211>    41
<212>    DNA
<213>    Artificial Sequence

<220>
<223>    Primer


<400>    7
gttcttccag attctctact tttcgagctc ggtacctacc g                          41


<210>    8
<211>    42
<212>    DNA
<213>    Artificial Sequence

<220>
<223>    Primer


<400>    8
tgaagaggtg tttattcaac tgggggtacc gttcgtataa tg                         42


<210>    9
<211>    21
<212>    DNA
<213>    Artificial Sequence

<220>
<223>    Primer


<400>    9
ataaccacca tcagttcgat a                                                21


<210>    10
<211>    41
<212>    DNA
<213>    Artificial Sequence

<220>
<223>    Primer


<400>    10
cggtaggtac cgagctcgaa aagtagagaa tctggaagaa c                          41


<210>    11
<211>    347
<212>    PRT
<213>    Escherichia coli

<400>    11

Met Ser Val Met Phe Asp Pro Asp Thr Ala Ile Tyr Pro Phe Pro Pro
1               5                   10                  15

Lys Pro Thr Pro Leu Ser Ile Asp Glu Lys Ala Tyr Tyr Arg Glu Lys
            20                  25                  30

Ile Lys Arg Leu Leu Lys Glu Arg Asn Ala Val Met Val Ala His Tyr
        35                  40                  45

Tyr Thr Asp Pro Glu Ile Gln Gln Leu Ala Glu Glu Thr Gly Gly Cys
    50                  55                  60

Ile Ser Asp Ser Leu Glu Met Ala Arg Phe Gly Ala Lys His Pro Ala
65                  70                  75                  80

Ser Thr Leu Leu Val Ala Gly Val Arg Phe Met Gly Glu Thr Ala Lys
                85                  90                  95

Ile Leu Ser Pro Glu Lys Thr Ile Leu Met Pro Thr Leu Gln Ala Glu
            100                 105                 110

Cys Ser Leu Asp Leu Gly Cys Pro Val Glu Glu Phe Asn Ala Phe Cys
        115                 120                 125

Asp Ala His Pro Asp Arg Thr Val Val Val Tyr Ala Asn Thr Ser Ala
    130                 135                 140

Ala Val Lys Ala Arg Ala Asp Trp Val Val Thr Ser Ser Ile Ala Val
145                 150                 155                 160

Glu Leu Ile Asp His Leu Asp Ser Leu Gly Glu Lys Ile Ile Trp Ala
                165                 170                 175

Pro Asp Lys His Leu Gly Arg Tyr Val Gln Lys Gln Thr Gly Gly Asp
            180                 185                 190

Ile Leu Cys Trp Gln Gly Ala Cys Ile Val His Asp Glu Phe Lys Thr
        195                 200                 205

Gln Ala Leu Thr Arg Leu Gln Glu Glu Tyr Pro Asp Ala Ala Ile Leu
    210                 215                 220

Val His Pro Glu Ser Pro Gln Ala Ile Val Asp Met Ala Asp Ala Val
225                 230                 235                 240

Gly Ser Thr Ser Gln Leu Ile Ala Ala Ala Lys Thr Leu Pro His Gln
                245                 250                 255

Arg Leu Ile Val Ala Thr Asp Arg Gly Ile Phe Tyr Lys Met Gln Gln
            260                 265                 270

Ala Val Pro Asp Lys Glu Leu Leu Glu Ala Pro Thr Ala Gly Glu Gly
        275                 280                 285

Ala Thr Cys Arg Ser Cys Ala His Cys Pro Trp Met Ala Met Asn Gly
    290                 295                 300

Leu Gln Ala Ile Ala Glu Ala Leu Glu Gln Glu Gly Ser Asn His Glu
305                 310                 315                 320

Val His Val Asp Glu Arg Leu Arg Glu Arg Ala Leu Val Pro Leu Asn
                325                 330                 335

Arg Met Leu Asp Phe Ala Ala Thr Leu Arg Gly
            340                 345

<210>    12
<211>    1044
<212>    DNA
<213>    Escherichia coli

<400>    12
   atgagcgtaa tgtttgatcc agacacggcg atttatcctt tccccccgaa gccgacgccg        60
   ttaagcattg atgaaaaagc gtattaccgc gagaagataa aacgtctgct aaaagaacgt       120
   aatgcggtga tggttgccca ctactatacc gatcccgaaa ttcaacaact ggcagaagaa       180
   accggtggct gtatttctga ttctctggaa atggcgcgct tcggtgcaaa gcatcccgct       240
   tctactttgt tagtcgctgg ggtgagattt atgggagaaa ccgccaaaat tctcagtccg       300
   gaaaaaacaa ttctgatgcc gacacttcag gctgaatgtt cactggatct cggctgccct       360
   gttgaagaat ttaacgcatt ttgcgatgcc catcccgatc gtactgtcgt cgtctacgcc       420
   aacacttctg ctgcggtaaa agcgcgcgca gattgggtgg taacttcaag cattgccgtc       480
   gaacttattg atcatcttga tagtttgggt gaaaaaatca tctgggcacc cgacaaacat       540
   ctggggcgtt acgtgcaaaa acagacgggt ggagacattc tatgctggca gggtgcctgt       600
   attgtgcatg atgaatttaa gactcaggcg ttaacccgct tgcaagaaga atacccggat       660
   gctgccatac tggtgcatcc agaatcacca caagctattg tcgatatggc ggatgcggtc       720
   ggttccacca gtcaactgat cgctgctgcg aaaacattgc cacatcagag gcttattgtg       780
   gcaaccgatc ggggtatttt ctacaaaatg cagcaggcgg tgccagataa agagttactg       840
   gaagcaccaa ccgcaggtga gggtgcaacc tgccgcagct gcgcgcattg tccgtggatg       900
   gccatgaatg gccttcaggc catcgcagag gcattagaac aggaaggaag caatcacgag       960
   gttcatgttg atgaaaggct gcgagagagg gcgctggtgc cgctcaatcg tatgctggat      1020
   tttgcggcta cactacgtgg ataa                                             1044

<210>    13
<211>    39
<212>    DNA
<213>    Artificial Sequence

<220>
<223>    Primer

<400>    13
cattatacga acggtacccc cccggatgct gccatactg                              39

<210>    14
<211>    20
<212>    DNA

<213>    Artificial Sequence

<220>
<223>    Primer


<400>    14
ccatgagaga tcataaccgc                                                    20


<210>    15
<211>    40
<212>    DNA
<213>    Artificial Sequence

<220>
<223>    Primer


<400>    15
tgacttttcc ccaccattcg ttcgagctcg gtacctaccg                              40


<210>    16
<211>    39
<212>    DNA
<213>    Artificial Sequence

<220>
<223>    Primer


<400>    16
cagtatggca gcatccgggg gggtaccgtt cgtataatg                               39


<210>    17
<211>    20
<212>    DNA
<213>    Artificial Sequence

<220>
<223>    Primer


<400>    17
gggtcgttag ctcagttggt                                                    20


<210>    18
<211>    40
<212>    DNA
<213>    Artificial Sequence

<220>
<223>    Primer


<400>    18
cggtaggtac cgagctcgaa cgaatggtgg ggaaaagtca                              40


<210>    19

<211>    540
<212>    PRT
<213>    Escherichia coli

<400>    19
Met Asn Thr Leu Pro Glu His Ser Cys Asp Val Leu Ile Ile Gly Ser
1               5                   10                  15

Gly Ala Ala Gly Leu Ser Leu Ala Leu Arg Leu Ala Asp Gln His Gln
            20                  25                  30

Val Ile Val Leu Ser Lys Gly Pro Val Thr Glu Gly Ser Thr Phe Tyr
        35                  40                  45

Ala Gln Gly Gly Ile Ala Ala Val Phe Asp Glu Thr Asp Ser Ile Asp
    50                  55                  60

Ser His Val Glu Asp Thr Leu Ile Ala Gly Ala Gly Ile Cys Asp Arg
65                  70                  75                  80

His Ala Val Glu Phe Val Ala Ser Asn Ala Arg Ser Cys Val Gln Trp
                85                  90                  95

Leu Ile Asp Gln Gly Val Leu Phe Asp Thr His Ile Gln Pro Asn Gly
            100                 105                 110

Glu Glu Ser Tyr His Leu Thr Arg Glu Gly Gly His Ser His Arg Arg
        115                 120                 125

Ile Leu His Ala Ala Asp Ala Thr Gly Arg Glu Val Glu Thr Thr Leu
    130                 135                 140

Val Ser Lys Ala Leu Asn His Pro Asn Ile Arg Val Leu Glu Arg Ser
145                 150                 155                 160

Asn Ala Val Asp Leu Ile Val Ser Asp Lys Ile Gly Leu Pro Gly Thr
                165                 170                 175

Arg Arg Val Val Gly Ala Trp Val Trp Asn Arg Asn Lys Glu Thr Val
            180                 185                 190

Glu Thr Cys His Ala Lys Ala Val Val Leu Ala Thr Gly Gly Ala Ser
        195                 200                 205

Lys Val Tyr Gln Tyr Thr Thr Asn Pro Asp Ile Ser Ser Gly Asp Gly
    210                 215                 220

Ile Ala Met Ala Trp Arg Ala Gly Cys Arg Val Ala Asn Leu Glu Phe
225                 230                 235                 240

Asn Gln Phe His Pro Thr Ala Leu Tyr His Pro Gln Ala Arg Asn Phe
                245                 250                 255

Leu Leu Thr Glu Ala Leu Arg Gly Glu Gly Ala Tyr Leu Lys Arg Pro
            260                 265                 270

Asp Gly Thr Arg Phe Met Pro Asp Phe Asp Glu Arg Gly Glu Leu Ala
        275                 280                 285

Pro Arg Asp Ile Val Ala Arg Ala Ile Asp His Glu Met Lys Arg Leu
    290                 295                 300

Gly Ala Asp Cys Met Phe Leu Asp Ile Ser His Lys Pro Ala Asp Phe

305                 310                 315                 320

Ile Arg Gln His Phe Pro Met Ile Tyr Glu Lys Leu Leu Gly Leu Gly
                325                 330                 335

Ile Asp Leu Thr Gln Glu Pro Val Pro Ile Val Pro Ala Ala His Tyr
            340                 345                 350

Thr Cys Gly Gly Val Met Val Asp Asp His Gly Arg Thr Asp Val Glu
        355                 360                 365

Gly Leu Tyr Ala Ile Gly Glu Val Ser Tyr Thr Gly Leu His Gly Ala
    370                 375                 380

Asn Arg Met Ala Ser Asn Ser Leu Leu Glu Cys Leu Val Tyr Gly Trp
385                 390                 395                 400

Ser Ala Ala Glu Asp Ile Thr Arg Arg Met Pro Tyr Ala His Asp Ile
                405                 410                 415

Ser Thr Leu Pro Pro Trp Asp Glu Ser Arg Val Glu Asn Pro Asp Glu
            420                 425                 430

Arg Val Val Ile Gln His Asn Trp His Glu Leu Arg Leu Phe Met Trp
        435                 440                 445

Asp Tyr Val Gly Ile Val Arg Thr Thr Lys Arg Leu Glu Arg Ala Leu
    450                 455                 460

Arg Arg Ile Thr Met Leu Gln Gln Glu Ile Asp Glu Tyr Tyr Ala His
465                 470                 475                 480

Phe Arg Val Ser Asn Asn Leu Leu Glu Leu Arg Asn Leu Val Gln Val
                485                 490                 495

Ala Glu Leu Ile Val Arg Cys Ala Met Met Arg Lys Glu Ser Arg Gly
            500                 505                 510

Leu His Phe Thr Leu Asp Tyr Pro Glu Leu Leu Thr His Ser Gly Pro
        515                 520                 525

Ser Ile Leu Ser Pro Gly Asn His Tyr Ile Asn Arg
    530                 535                 540

<210>    20
<211>    1623
<212>    DNA
<213>    Escherichia coli

<400>    20
atgaatactc tccctgaaca ttcatgtgac gtgttgatta tcggtagcgg cgcagccgga      60

ctttcactgg cgctacgcct ggctgaccag catcaggtca tcgttctaag taaaggcccg     120

gtaacggaag gttcaacatt ttatgcccag ggcggtattg ccgccgtgtt tgatgaaact     180

gacagcattg actcgcatgt ggaagacaca ttgattgccg gggctggtat ttgcgatcgc     240

catgcagttg aatttgtcgc cagcaatgca cgatcctgtg tgcaatggct aatcgaccag     300

ggggtgttgt ttgataccca cattcaaccg aatggcgaag aaagttacca tctgacccgt     360

gaaggtggac atagtcaccg tcgtattctt catgccgccg acgccaccgg tagagaagta 420

gaaaccacgc tggtgagcaa ggcgctgaac catccgaata ttcgcgtgct ggagcgcagc 480

aacgcggttg atctgattgt ttctgacaaa attggcctgc cgggcacgcg acgggttgtt 540

ggcgcgtggg tatggaaccg taataaagaa acggtggaaa cctgccacgc aaaagcggtg 600

gtgctggcaa ccggcggtgc gtcgaaggtt tatcagtaca ccaccaatcc ggatatttct 660

tctggcgatg gcattgctat ggcgtggcgc gcaggctgcc gggttgccaa tctcgaattt 720

aatcagttcc accctaccgc gctatatcac ccacaggcac gcaatttcct gttaacagaa 780

gcactgcgcg gcgaaggcgc ttatctcaag cgcccggatg gtacgcgttt tatgcccgat 840

tttgatgagc gcggcgaact ggccccgcgc gatattgtcg cccgcgccat tgaccatgaa 900

atgaaacgcc tcggcgcaga ttgtatgttc cttgatatca gccataagcc cgccgatttt 960

attcgccagc atttcccgat gatttatgaa aagctgctcg ggctggggat tgatctcaca 1020

caagaaccgg taccgattgt gcctgctgca cattatacct gcggtggtgt aatggttgat 1080

gatcatgggc gtacggacgt cgagggcttg tatgccattg gcgaggtgag ttataccggc 1140

ttacacggcg ctaaccgcat ggcctcgaat tcattgctgg agtgtctggt ctatggctgg 1200

tcggcggcgg aagatatcac cagacgtatg ccttatgccc acgacatcag tacgttaccg 1260

ccgtgggatg aaagccgcgt tgagaaccct gacgaacggg tagtaattca gcataactgg 1320

cacgagctac gtctgtttat gtgggattac gttggcattg tgcgcacaac gaagcgcctg 1380

gaacgcgccc tgcggcggat aaccatgctc caacaagaaa tagacgaata ttacgcccat 1440

ttccgcgtct caaataattt gctggagctg cgtaatctgg tacaggttgc cgagttgatt 1500

gttcgctgtg caatgatgcg taaagagagt cgggggttgc atttcacgct ggattatccg 1560

gaactgctca cccattccgg tccgtcgatc ctttcccccg gcaatcatta cataaacaga 1620

taa 1623

<210> 21
<211> 29
<212> DNA
<213> Artificial Sequence

<220>
<223> Primer


<400> 21
aattcatatg aatactctcc ctgaacatt 29


<210> 22
<211> 32
<212> DNA
<213> Artificial Sequence

<220>
<223>    Primer


<400>    22
aattggatcc ctataccact acgcttgatc ac                                    32


<210>    23
<211>    23
<212>    DNA
<213>    Artificial Sequence

<220>
<223>    Primer


<400>    23
ctgcagatcc tgcacgaccc acc                                              23


<210>    24
<211>    23
<212>    DNA
<213>    Artificial Sequence

<220>
<223>    Primer


<400>    24
tctagactta ccatctcgtt tta                                              23


<210>    25
<211>    23
<212>    DNA
<213>    Artificial Sequence

<220>
<223>    Primer


<400>    25
tctagaatga gcgtaatgtt tga                                              23


<210>    26
<211>    23
<212>    DNA
<213>    Artificial Sequence

<220>
<223>    Primer


<400>    26
ggatccttat ccacgtagtg tag                                              23


<210>    27
<211>    1356
<212>    DNA

<213>    Artificial Sequence

<220>
<223>    pNadA-nadA


<400>    27
ctgcagatcc tgcacgaccc accaatgtaa aaaagcgccc taaaggcgct tttttgctat    60

tcaggcatcc tcaatttcac tttgtaaacc tgatgacatc gtcagagctt actgtgcaag    120

caactctatg tcggtggaat taggcgtaaa atgacgcatc ctgcacatta ggcgtaattc    180

gagtgacttt tccccaccat tcgactatct tgtttagcat ataaaacaaa ttacaccgat    240

aacagcgaat attacgctaa tgtcggtttt aacgttaagc ctgtaaaacg agatggtaag    300

tctagaatga gcgtaatgtt tgatccagac acggcgattt atcctttccc cccgaagccg    360

acgccgttaa gcattgatga aaaagcgtat taccgcgaga agataaaacg tctgctaaaa    420

gaacgtaatg cggtgatggt tgcccactac tataccgatc ccgaaattca acaactggca    480

gaagaaaccg gtggctgtat ttctgattct ctggaaatgg cgcgcttcgg tgcaaagcat    540

cccgcttcta ctttgttagt cgctggggtg agatttatgg gagaaaccgc caaaattctc    600

agtccggaaa aaacaattct gatgccgaca cttcaggctg aatgttcact ggatctcggc    660

tgccctgttg aagaatttaa cgcattttgc gatgcccatc ccgatcgtac tgtcgtcgtc    720

tacgccaaca cttctgctgc ggtaaaagcg cgcgcagatt gggtggtaac ttcaagcatt    780

gccgtcgaac ttattgatca tcttgatagt ttgggtgaaa aaatcatctg ggcacccgac    840

aaacatctgg ggcgttacgt gcaaaaacag acgggtggag acattctatg ctggcagggt    900

gcctgtattg tgcatgatga atttaagact caggcgttaa cccgcttgca agaagaatac    960

ccggatgctg ccatactggt gcatccagaa tcaccacaag ctattgtcga tatggcggat    1020

gcggtcggtt ccaccagtca actgatcgct gctgcgaaaa cattgccaca tcagaggctt    1080

attgtggcaa ccgatcgggg tattttctac aaaatgcagc aggcggtgcc agataaagag    1140

ttactggaag caccaaccgc aggtgagggt gcaacctgcc gcagctgcgc gcattgtccg    1200

tggatggcca tgaatggcct tcaggccatc gcagaggcat tagaacagga aggaagcaat    1260

cacgaggttc atgttgatga aaggctgcga gagagggcgc tggtgccgct caatcgtatg    1320

ctggattttg cggctacact acgtggataa ggatcc    1356


<210>    28
<211>    23
<212>    DNA
<213>    Artificial Sequence

<220>
<223>    primer

<400>    28
tctagaatga gcgtaatgtt tga                                              23


<210>    29
<211>    23
<212>    DNA
<213>    Artificial Sequence

<220>
<223>    primer


<400>    29
ggatccttat ccccgtagtg tag                                              23


<210>    30
<211>    1356
<212>    DNA
<213>    Artificial Sequence

<220>
<223>    pNadA-nadA(KP)


<400>    30
ctgcagatcc tgcacgaccc accaatgtaa aaaagcgccc taaaggcgct tttttgctat      60

tcaggcatcc tcaatttcac tttgtaaacc tgatgacatc gtcagagctt actgtgcaag     120

caactctatg tcggtggaat taggcgtaaa atgacgcatc ctgcacatta ggcgtaattc     180

gagtgacttt tccccaccat tcgactatct tgtttagcat ataaaacaaa ttacaccgat     240

aacagcgaat attacgctaa tgtcggtttt aacgttaagc ctgtaaaacg agatggtaag     300

tctagaatga gcgtaatgtt tgatcctgaa acggcgattt atcctttccc tgctaaaccg     360

cagccgctga ccgtcgacga aaagcagttt taccgcgaaa aaatcaagcg cctgctgcgc     420

gagcgcgatg ccgtgatggt ggcgcattac tacaccgatc ctgaaattca acagctggcg     480

gaagagaccg gcggctgtat cgccgactcg ctggagatgg cgcgctttgg cgcccgccat     540

tcggcctcca cgctgctggt cgccggggtg cgttttatgg gggaaaccgc caaaattctc     600

agcccggaaa agaccatttt gatgccgacc ctgaacgccg agtgttcatt ggatctgggc     660

tgtccgattg aggaattcaa cgccttttgc gacgcccatc ctgaccgcac cgtcgtggtc     720

tatgccaata cgtccgccgc ggtgaaggcc cgcgccgact gggtggtgac ctccagcatc     780

gccgtggaac tcattgacca tctggatagt cttggtcaaa agatcctctg ggcgccggac     840

cgccaccttg ggcgttacgt tcagcgtcag accggcgcag acgtgctgtg ctggcagggg     900

gcgtgcatcg tgcacgacga gtttaaaacc caggcgttga tgcggatgaa ggctctgcat     960

cccgaagccg ccgtgctggt ccatcccgag tcgccgcagg cgatcgttga gatggccgat    1020

gccgtaggct ccaccagcca gctgattgcg gcggcgaaaa gcctgcccca gcgccagctg    1080

atcgtggcca ccgatcgcgg tattttctat aaaatgcagc aggcggtgcc ggagaaaacg 1140

ctgctggaag cgcccaccgc cggcgaaggg gcgacctgcc gcagctgcgc gcattgtccg 1200

tggatggcga tgaatggcct gaaagccatt gccgaggggc tcgagcaggg cggcgctgaa 1260

catgaaatcc atgtcgacga agcgctgcga accggcgcat taattcccct taaccggatg 1320

ctggattttg cggctacact acggggataa ggatcc 1356

<210> 31
<211> 31
<212> DNA
<213> Artificial Sequence

<220>
<223> primer

<400> 31
aattgggccc atgagcgtaa tgtttgatcc a 31

<210> 32
<211> 29
<212> DNA
<213> Artificial Sequence

<220>
<223> primer

<400> 32
aattgcggcc gctcgtgcct accgcttcg 29

<210> 33
<211> 32
<212> DNA
<213> Artificial Sequence

<220>
<223> primer

<400> 33
ccgcggatcc caccgcgggc ttattccatt ac 32

<210> 34
<211> 34
<212> DNA
<213> Artificial Sequence

<220>
<223> primer

<400> 34
gatgggccca tcttaatctc ctagattggg tttc 34

<210> 35
<211> 31
<212> DNA
<213> Artificial Sequence

<220>
<223> primer

<400> 35
aattgggccc atgagcgtaa tgtttgatcc t 31

<210> 36
<211> 29
<212> DNA
<213> Artificial Sequence

<220>
<223> primer

<400> 36
aattgcggcc gctcgtcccc acggcctct 29

<210> 37
<211> 3096
<212> DNA
<213> Escherichia coli

<400> 37
ataaggcgct cgcgccgcat ccggcactgt tgccaaactc cagtgccgca ataatgtcgg 60

atgcgatact tgcgcatctt atccgaccta cacctttggt gttacttggg gcgatttttt 120

aacatttcca taagttacgc ttatttaaag cgtcgtgaat ttaatgacgt aaattcctgc 180

tatttattcg tttgctgaag cgatttcgca gcatttgacg tcaccgcttt tacgtggctt 240

tataaaagac gacgaaaagc aaagcccgag catattcgcg ccaatgcgac gtgaaggata 300

cagggctatc aaacgataag atggggtgtc tggggtaata tgaacgaaca atattccgca 360

ttgcgtagta atgtcagtat gctcggcaaa gtgctgggag aaaccatcaa ggatgcgttg 420

ggagaacaca ttcttgaacg cgtagaaact atccgtaagt tgtcgaaatc ttcacgcgct 480

ggcaatgatg ctaaccgcca ggagttgctc accaccttac aaaatttgtc gaacgacgag 540

ctgctgcccg ttgcgcgtgc gtttagtcag ttcctgaacc tggccaacac cgccgagcaa 600

taccacagca tttcgccgaa aggcgaagct gccagcaacc cggaagtgat cgcccgcacc 660

ctgcgtaaac tgaaaaacca gccggaactg agcgaagaca ccatcaaaaa agcagtggaa 720

tcgctgtcgc tggaactggt cctcacggct cacccaaccg aaattacccg tcgtacactg 780

atccacaaaa tggtggaagt gaacgcctgt ttaaaacagc tcgataacaa agatatcgct 840

gactacgaac acaaccagct gatgcgtcgc ctgcgccagt tgatcgccca gtcatggcat 900

accgatgaaa tccgtaagct gcgtccaagc ccggtagatg aagccaaatg gggctttgcc 960

gtagtggaaa acagcctgtg gcaaggcgta ccaaattacc tgcgcgaact gaacgaacaa 1020

ctggaagaga acctcggcta caaactgccc gtcgaatttg ttccggtccg ttttacttcg 1080

tggatgggcg gcgaccgcga cggcaacccg aacgtcactg ccgatatcac ccgccacgtc 1140

ctgctactca gccgctggaa agccaccgat ttgttcctga aagatattca ggtgctggtt 1200

tctgaactgt cgatggttga agcgacccct gaactgctgg cgctggttgg cgaagaaggt 1260

gccgcagaac cgtatcgcta tctgatgaaa aacctgcgtt ctcgcctgat ggcgacacag 1320

gcatggctgg aagcgcgcct gaaaggcgaa gaactgccaa aaccagaagg cctgctgaca 1380

caaaacgaag aactgtggga accgctctac gcttgctacc agtcacttca ggcgtgtggc 1440

atgggtatta tcgccaacgg cgatctgctc gacaccctgc gccgcgtgaa atgtttcggc 1500

gtaccgctgg tccgtattga tatccgtcag gagagcacgc gtcataccga agcgctgggc 1560

gagctgaccc gctacctcgg tatcggcgac tacgaaagct ggtcagaggc cgacaaacag 1620

gcgttcctga tccgcgaact gaactccaaa cgtccgcttc tgccgcgcaa ctggcaacca 1680

agcgccgaaa cgcgcgaagt gctcgatacc tgccaggtga ttgccgaagc accgcaaggc 1740

tccattgccg cctacgtgat ctcgatggcg aaaacgccgt ccgacgtact ggctgtccac 1800

ctgctgctga aagaagcggg tatcgggttt gcgatgccgg ttgctccgct gtttgaaacc 1860

ctcgatgatc tgaacaacgc caacgatgtc atgacccagc tgctcaatat tgactggtat 1920

cgtggcctga ttcagggcaa acagatggtg atgattggct attccgactc agcaaaagat 1980

gcgggagtga tggcagcttc ctgggcgcaa tatcaggcac aggatgcatt aatcaaaacc 2040

tgcgaaaaag cgggtattga gctgacgttg ttccacggtc gcggcggttc cattggtcgc 2100

ggcggcgcac ctgctcatgc ggcgctgctg tcacaaccgc caggaagcct gaaaggcggc 2160

ctgcgcgtaa ccgaacaggg cgagatgatc cgctttaaat atggtctgcc agaaatcacc 2220

gtcagcagcc tgtcgcttta taccggggcg attctggaag ccaacctgct gccaccgccg 2280

gagccgaaag agagctggcg tcgcattatg gatgaactgt cagtcatctc ctgcgatgtc 2340

taccgcggct acgtacgtga aaacaaagat tttgtgcctt acttccgctc cgctacgccg 2400

gaacaagaac tgggcaaact gccgttgggt tcacgtccgg cgaaacgtcg cccaaccggc 2460

ggcgtcgagt cactacgcgc cattccgtgg atcttcgcct ggacgcaaaa ccgtctgatg 2520

ctccccgcct ggctgggtgc aggtacggcg ctgcaaaaag tggtcgaaga cggcaaacag 2580

agcgagctgg aggctatgtg ccgcgattgg ccattcttct cgacgcgtct cggcatgctg 2640

gagatggtct tcgccaaagc agacctgtgg ctggcggaat actatgacca acgcctggta 2700

gacaaagcac tgtggccgtt aggtaaagag ttacgcaacc tgcaagaaga agacatcaaa 2760

gtggtgctgg cgattgccaa cgattcccat ctgatggccg atctgccgtg gattgcagag 2820

tctattcagc tacggaatat ttacaccgac ccgctgaacg tattgcaggc cgagttgctg 2880

caccgctccc gccaggcaga aaaagaaggc caggaaccgg atcctcgcgt cgaacaagcg 2940

ttaatggtca ctattgccgg gattgcggca ggtatgcgta ataccggcta atcttcctct 3000

tctgcaaacc ctcgtgcttt tgcgcgaggg ttttctgaaa tacttctgtt ctaacaccct 3060

cgttttcaat atatttctgt ctgcatttta ttcaaa 3096

<210> 38
<211> 37
<212> DNA
<213> Artificial Sequence

<220>
<223> primer

<400> 38
aagcttctgt aggccggata aggcgctcgc gccgcat 37

<210> 39
<211> 27
<212> DNA
<213> Artificial Sequence

<220>
<223> primer

<400> 39
cggatccttt gaataaaatg cagacag 27

<210> 40
<211> 1556
<212> DNA
<213> Escherichia coli

<400> 40
gtccacctat gttgactaca tcatcaacca gatcgattct gacaacaaac tgggcgtagg 60

ttcagacgac accgttgctg tgggtatcgt ttaccagttc taatagcaca cctctttgtt 120

aaatgccgaa aaaacaggac tttggtcctg ttttttttat accttccaga gcaatctcac 180

gtcttgcaaa aacagcctgc gttttcatca gtaatagttg gaattttgta aatctcccgt 240

taccctgata gcggacttcc cttctgtaac cataatggaa cctcgtcatg tttgagaaca 300

ttaccgccgc tcctgccgac ccgattctgg gcctggccga tctgtttcgt gccgatgaac 360

gtcccggcaa aattaacctc gggattggtg tctataaaga tgagacgggc aaaaccccgg 420

tactgaccag cgtgaaaaag gctgaacagt atctgctcga aaatgaaacc accaaaaatt 480

acctcggcat tgacggcatc cctgaatttg gtcgctgcac tcaggaactg ctgtttggta 540

aaggtagcgc cctgatcaat gacaaacgtg ctcgcacggc acagactccg gggggcactg 600

gcgcactacg cgtggctgcc gatttcctgg caaaaaatac cagcgttaag cgtgtgtggg 660

tgagcaaccc aagctggccg aaccataaga gcgtctttaa ctctgcaggt ctggaagttc 720

gtgaatacgc ttattatgat gcggaaaatc acactcttga cttcgatgca ctgattaaca 780

gcctgaatga agctcaggct ggcgacgtag tgctgttcca tggctgctgc cataacccaa 840

ccggtatcga ccctacgctg gaacaatggc aaacactggc acaactctcc gttgagaaag 900

gctggttacc gctgtttgac ttcgcttacc agggttttgc ccgtggtctg gaagaagatg 960

ctgaaggact gcgcgctttc gcggctatgc ataaagagct gattgttgcc agttcctact 1020

ctaaaaactt tggcctgtac aacgagcgtg ttggcgcttg tactctggtt gctgccgaca 1080

gtgaaaccgt tgatcgcgca ttcagccaaa tgaaagcggc gattcgcgct aactactcta 1140

acccaccagc acacggcgct tctgttgttg ccaccatcct gagcaacgat gcgttacgtg 1200

cgatttggga acaagagctg actgatatgc gccagcgtat tcagcgtatg cgtcagttgt 1260

tcgtcaatac gctgcaggaa aaaggcgcaa accgcgactt cagctttatc atcaaacaga 1320

acggcatgtt ctccttcagt ggcctgacaa aagaacaagt gctgcgtctg cgcgaagagt 1380

ttggcgtata tgcggttgct tctggtcgcg taaatgtggc cgggatgaca ccagataaca 1440

tggctccgct gtgcgaagcg attgtggcag tgctgtaagc attaaaaaca atgaagcccg 1500

ctgaaaagcg ggctgagact gatgacaaac gcaacattgc ctgatgcgct acgctt 1556

<210> 41
<211> 26
<212> DNA
<213> Artificial Sequence

<220>
<223> primer

<400> 41
ggatccgtcc acctatgttg actaca 26

<210> 42
<211> 31
<212> DNA
<213> Artificial Sequence

<220>
<223> primer

<400> 42
ggtaccgagc tcataagcgt agcgcatcag g 31

<210> 43
<211> 9193
<212> DNA
<213> Artificial Sequence

<220>
<223>    pCPA

<400>    43
cccgtcttac tgtcgggaat tcgcgttggc cgattcatta atgcagctgg cacgacaggt      60

ttcccgactg gaaagcgggc agtgagcgca acgcaattaa tgtgagttag ctcactcatt     120

aggcacccca ggctttacac tttatgcttc cggctcgtat gttgtgtgga attgtgagcg     180

gataacaatt tcacacagga aacagctatg accatgatta cgccaagctt ctgtaggccg     240

gataaggcgc tcgcgccgca tccggcactg ttgccaaact ccagtgccgc aataatgtcg     300

gatgcgatac ttgcgcatct tatccgacct acacctttgg tgttacttgg ggcgattttt     360

taacatttcc ataagttacg cttatttaaa gcgtcgtgaa tttaatgacg taaattcctg     420

ctatttattc gtttgctgaa gcgatttcgc agcatttgac gtcaccgctt ttacgtggct     480

ttataaaaga cgacgaaaag caaagcccga gcatattcgc gccaatgcga cgtgaaggat     540

acagggctat caaacgataa gatggggtgt ctggggtaat atgaacgaac aatattccgc     600

attgcgtagt aatgtcagta tgctcggcaa agtgctggga gaaaccatca aggatgcgtt     660

gggagaacac attcttgaac gcgtagaaac tatccgtaag ttgtcgaaat cttcacgcgc     720

tggcaatgat gctaaccgcc aggagttgct caccacctta caaaatttgt cgaacgacga     780

gctgctgccc gttgcgcgtg cgtttagtca gttcctgaac ctggccaaca ccgccgagca     840

ataccacagc atttcgccga aaggcgaagc tgccagcaac ccggaagtga tcgcccgcac     900

cctgcgtaaa ctgaaaaacc agccggaact gagcgaagac accatcaaaa aagcagtgga     960

atcgctgtcg ctggaactgg tcctcacggc tcacccaacc gaaattaccc gtcgtacact    1020

gatccacaaa atggtggaag tgaacgcctg tttaaaacag ctcgataaca aagatatcgc    1080

tgactacgaa cacaaccagc tgatgcgtcg cctgcgccag ttgatcgccc agtcatggca    1140

taccgatgaa atccgtaagc tgcgtccaag cccggtagat gaagccaaat ggggctttgc    1200

cgtagtggaa aacagcctgt ggcaaggcgt accaaattac ctgcgcgaac tgaacgaaca    1260

actggaagag aacctcggct acaaactgcc cgtcgaattt gttccggtcc gttttacttc    1320

gtggatgggc ggcgaccgcg acggcaaccc gaacgtcact gccgatatca cccgccacgt    1380

cctgctactc agccgctgga aagccaccga tttgttcctg aaagatattc aggtgctggt    1440

ttctgaactg tcgatggttg aagcgacccc tgaactgctg gcgctggttg gcgaagaagg    1500

tgccgcagaa ccgtatcgct atctgatgaa aaacctgcgt tctcgcctga tggcgacaca    1560

ggcatggctg gaagcgcgcc tgaaaggcga agaactgcca aaaccagaag gcctgctgac    1620

acaaaacgaa gaactgtggg aaccgctcta cgcttgctac cagtcacttc aggcgtgtgg    1680

catgggtatt atcgccaacg gcgatctgct cgacaccctg cgccgcgtga aatgtttcgg    1740

cgtaccgctg gtccgtattg atatccgtca ggagagcacg cgtcataccg aagcgctggg 1800

cgagctgacc cgctacctcg gtatcggcga ctacgaaagc tggtcagagg ccgacaaaca 1860

ggcgttcctg atccgcgaac tgaactccaa acgtccgctt ctgccgcgca actggcaacc 1920

aagcgccgaa acgcgcgaag tgctcgatac ctgccaggtg attgccgaag caccgcaagg 1980

ctccattgcc gcctacgtga tctcgatggc gaaaacgccg tccgacgtac tggctgtcca 2040

cctgctgctg aaagaagcgg gtatcgggtt tgcgatgccg gttgctccgc tgtttgaaac 2100

cctcgatgat ctgaacaacg ccaacgatgt catgacccag ctgctcaata ttgactggta 2160

tcgtggcctg attcagggca aacagatggt gatgattggc tattccgact cagcaaaaga 2220

tgcgggagtg atggcagctt cctgggcgca atatcaggca caggatgcat taatcaaaac 2280

ctgcgaaaaa gcgggtattg agctgacgtt gttccacggt cgcggcggtt ccattggtcg 2340

cggcggcgca cctgctcatg cggcgctgct gtcacaaccg ccaggaagcc tgaaaggcgg 2400

cctgcgcgta accgaacagg gcgagatgat ccgctttaaa tatggtctgc cagaaatcac 2460

cgtcagcagc ctgtcgcttt ataccggggc gattctggaa gccaacctgc tgccaccgcc 2520

ggagccgaaa gagagctggc gtcgcattat ggatgaactg tcagtcatct cctgcgatgt 2580

ctaccgcggc tacgtacgtg aaaacaaaga ttttgtgcct tacttccgct ccgctacgcc 2640

ggaacaagaa ctgggcaaac tgccgttggg ttcacgtccg gcgaaacgtc gcccaaccgg 2700

cggcgtcgag tcactacgcg ccattccgtg gatcttcgcc tggacgcaaa accgtctgat 2760

gctccccgcc tggctgggtg caggtacggc gctgcaaaaa gtggtcgaag acggcaaaca 2820

gagcgagctg gaggctatgt gccgcgattg gccattcttc tcgacgcgtc tcggcatgct 2880

ggagatggtc ttcgccaaag cagacctgtg gctggcggaa tactatgacc aacgcctggt 2940

agacaaagca ctgtggccgt taggtaaaga gttacgcaac ctgcaagaag aagacatcaa 3000

agtggtgctg gcgattgcca acgattccca tctgatggcc gatctgccgt ggattgcaga 3060

gtctattcag ctacggaata tttacaccga cccgctgaac gtattgcagg ccgagttgct 3120

gcaccgctcc cgccaggcag aaaaagaagg ccaggaaccg gaccctcgcg tcgaacaagc 3180

gttaatggtc actattgccg ggattgcggc aggtatgcgt aataccggct aatcttcctc 3240

ttctgcaaac cctcgtgctt ttgcgcgagg gttttctgaa atacttctgt tctaacaccc 3300

tcgttttcaa tatatttctg tctgcatttt attcaaagga tccgtccacc tatgttgact 3360

acatcatcaa ccagatcgat tctgacaaca aactgggcgt aggttcagac gacaccgttg 3420

ctgtgggtat cgtttaccag ttctaatagc acacctcttt gttaaatgcc gaaaaaacag 3480

gactttggtc ctgttttttt tataccttcc agagcaatct cacgtcttgc aaaaacagcc 3540

tgcgttttca tcagtaatag ttggaatttt gtaaatctcc cgttaccctg atagcggact 3600

tcccttctgt aaccataatg gaacctcgtc atgtttgaga acattaccgc cgctcctgcc 3660

gacccgattc tgggcctggc cgatctgttt cgtgccgatg aacgtcccgg caaaattaac 3720

ctcgggattg gtgtctataa agatgagacg ggcaaaaccc cggtactgac cagcgtgaaa 3780

aaggctgaac agtatctgct cgaaaatgaa accaccaaaa attacctcgg cattgacggc 3840

atccctgaat ttggtcgctg cactcaggaa ctgctgtttg gtaaaggtag cgccctgatc 3900

aatgacaaac gtgctcgcac ggcacagact ccggggggca ctggcgcact acgcgtggct 3960

gccgatttcc tggcaaaaaa taccagcgtt aagcgtgtgt gggtgagcaa cccaagctgg 4020

ccgaaccata agagcgtctt taactctgca ggtctggaag ttcgtgaata cgcttattat 4080

gatgcggaaa atcacactct tgacttcgat gcactgatta acagcctgaa tgaagctcag 4140

gctggcgacg tagtgctgtt ccatggctgc tgccataacc caaccggtat cgaccctacg 4200

ctggaacaat ggcaaacact ggcacaactc tccgttgaga aaggctggtt accgctgttt 4260

gacttcgctt accagggttt tgcccgtggt ctggaagaag atgctgaagg actgcgcgct 4320

ttcgcggcta tgcataaaga gctgattgtt gccagttcct actctaaaaa ctttggcctg 4380

tacaacgagc gtgttggcgc ttgtactctg gttgctgccg acagtgaaac cgttgatcgc 4440

gcattcagcc aaatgaaagc ggcgattcgc gctaactact ctaacccacc agcacacggc 4500

gcttctgttg ttgccaccat cctgagcaac gatgcgttac gtgcgatttg ggaacaagag 4560

ctgactgata tgcgccagcg tattcagcgt atgcgtcagt tgttcgtcaa tacgctgcag 4620

gaaaaaggcg caaaccgcga cttcagcttt atcatcaaac agaacggcat gttctccttc 4680

agtggcctga caaaagaaca agtgctgcgt ctgcgcgaag agtttggcgt atatgcggtt 4740

gcttctggtc gcgtaaatgt ggccgggatg acaccagata acatggctcc gctgtgcgaa 4800

gcgattgtgg cagtgctgta agcattaaaa acaatgaagc ccgctgaaaa gcgggctgag 4860

actgatgaca aacgcaacat tgcctgatgc gctacgctta tgagctcggt accgagctcg 4920

aattcactgg ccgtcgtttt acaacgtcgt gactgggaaa accctggcgt tacccaactt 4980

aatcgccttg cagcacatcc ccctttcgcc agctggcgta atagcgaaga ggcccgcacc 5040

gatcgccctt cccaacagtt gcgcagcctg aatggcgaat ggcgcctgat gcggtatttt 5100

ctccttacgc atctgtgcgg tatttcacac cgcatatggt gcactctcag tacaatctgc 5160

tctgatgccg catagttaag ccagccccga cacccgccaa cacccgctga cgagcttagt 5220

aaagccctcg ctagatttta atgcggatgt tgcgattact tcgccaacta ttgcgataac 5280

aagaaaaagc cagcctttca tgatatatct cccaatttgt gtagggctta ttatgcacgc 5340

ttaaaaataa taaaagcaga cttgacctga tagtttggct gtgagcaatt atgtgcttag 5400

tgcatctaac gcttgagtta agccgcgccg cgaagcggcg tcggcttgaa cgaattgtta 5460

gacattattt gccgactacc ttggtgatct cgcctttcac gtagtggaca aattcttcca 5520

actgatctgc gcgcgaggcc aagcgatctt cttcttgtcc aagataagcc tgtctagctt 5580

caagtatgac gggctgatac tgggccggca ggcgctccat tgcccagtcg gcagcgacat 5640

ccttcggcgc gattttgccg gttactgcgc tgtaccaaat gcgggacaac gtaagcacta 5700

catttcgctc atcgccagcc cagtcgggcg gcgagttcca tagcgttaag gtttcattta 5760

gcgcctcaaa tagatcctgt tcaggaaccg gatcaaagag ttcctccgcc gctggaccta 5820

ccaaggcaac gctatgttct cttgcttttg tcagcaagat agccagatca atgtcgatcg 5880

tggctggctc gaagatacct gcaagaatgt cattgcgctg ccattctcca aattgcagtt 5940

cgcgcttagc tggataacgc cacggaatga tgtcgtcgtg cacaacaatg gtgacttcta 6000

cagcgcggag aatctcgctc tctccagggg aagccgaagt ttccaaaagg tcgttgatca 6060

aagctcgccg cgttgtttca tcaagcctta cggtcaccgt aaccagcaaa tcaatatcac 6120

tgtgtggctt caggccgcca tccactgcgg agccgtacaa atgtacggcc agcaacgtcg 6180

gttcgagatg gcgctcgatg acgccaacta cctctgatag ttgagtcgat acttcggcga 6240

tcaccgcttc cctcatgatg tttaactttg ttttagggcg actgccctgc tgcgtaacat 6300

cgttgctgct ccataacatc aaacatcgac ccacggcgta acgcgcttgc tgcttggatg 6360

cccgaggcat agactgtacc ccaaaaaaac agtcataaca agccatgaaa accgccactg 6420

cgccgttacc accgctgcgt tcggtcaagg ttctggacca gttgcgtgag cgcatacgct 6480

acttgcatta cagcttacga accgaacagg cttatgtcca ctgggttcgt gccttcatcc 6540

gtttccacgg tgtgcgtcac ccggcaacct tgggcagcag cgaagtcgag gcatttctgt 6600

cctggctggc gaacgagcgc aaggtttcgg tctccacgca tcgtcaggca ttggcggcct 6660

tgctgttctt ctacggcaag gtgctgtgca cggatctgcc ctggcttcag gagatcggaa 6720

gacctcggcc gtcgcggcgc ttgccggtgg tgctgacccc ggatgaagtg gttcgcatcc 6780

tcggttttct ggaaggcgag catcgtttgt tcgcccagct tctgtatgga acgggcatgc 6840

ggatcagtga gggtttgcaa ctgcgggtca aggatctgga tttcgatcac ggcacgatca 6900

tcgtgcggga gggcaagggc tccaaggatc gggccttgat gttacccgag agcttggcac 6960

ccagcctgcg cgagcagggg aattaattcc cacgggtttt gctgcccgca aacgggctgt 7020

tctggtgttg ctagtttgtt atcagaatcg cagatccggc ttcagccggt ttgccggctg 7080

aaagcgctat ttcttccaga attgccatga ttttttcccc acgggaggcg tcactggctc 7140

ccgtgttgtc ggcagctttg attcgataag cagcatcgcc tgtttcaggc tgtctatgtg 7200

tgactgttga gctgtaacaa gttgtctcag gtgttcaatt tcatgttcta gttgctttgt 7260

tttactggtt tcacctgttc tattaggtgt tacatgctgt tcatctgtta cattgtcgat 7320

ctgttcatgg tgaacagctt tgaatgcacc aaaaactcgt aaaagctctg atgtatctat 7380

cttttttaca ccgttttcat ctgtgcatat ggacagtttt ccctttgata tgtaacggtg 7440

aacagttgtt ctacttttgt ttgttagtct tgatgcttca ctgatagata caagagccat 7500

aagaacctca gatccttccg tatttagcca gtatgttctc tagtgtggtt cgttgttttt 7560

gcgtgagcca tgagaacgaa ccattgagat catacttact ttgcatgtca ctcaaaaatt 7620

ttgcctcaaa actggtgagc tgaatttttg cagttaaagc atcgtgtagt gtttttctta 7680

gtccgttatg taggtaggaa tctgatgtaa tggttgttgg tattttgtca ccattcattt 7740

ttatctggtt gttctcaagt tcggttacga gatccatttg tctatctagt tcaacttgga 7800

aaatcaacgt atcagtcggg cggcctcgct tatcaaccac caatttcata ttgctgtaag 7860

tgtttaaatc tttacttatt ggtttcaaaa cccattggtt aagcctttta aactcatggt 7920

agttattttc aagcattaac atgaacttaa attcatcaag gctaatctct atatttgcct 7980

tgtgagtttt cttttgtgtt agttctttta ataaccactc ataaatcctc atagagtatt 8040

tgttttcaaa agacttaaca tgttccagat tatattttat gaattttttt aactggaaaa 8100

gataaggcaa tatctcttca ctaaaaacta attctaattt ttcgcttgag aacttggcat 8160

agtttgtcca ctggaaaatc tcaaagcctt taaccaaagg attcctgatt tccacagttc 8220

tcgtcatcag ctctctggtt gctttagcta atacaccata agcattttcc ctactgatgt 8280

tcatcatctg agcgtattgg ttataagtga acgataccgt ccgttctttc cttgtagggt 8340

tttcaatcgt ggggttgagt agtgccacac agcataaaat tagcttggtt tcatgctccg 8400

ttaagtcata gcgactaatc gctagttcat ttgctttgaa aacaactaat tcagacatac 8460

atctcaattg gtctaggtga ttttaatcac tataccaatt gagatgggct agtcaatgat 8520

aattactagt ccttttcctt tgagttgtgg gtatctgtaa attctgctag acctttgctg 8580

gaaaacttgt aaattctgct agaccctctg taaattccgc tagacctttg tgtgtttttt 8640

ttgtttatat tcaagtggtt ataatttata gaataaagaa agaataaaaa aagataaaaa 8700

gaatagatcc cagccctgtg tataactcac tactttagtc agttccgcag tattacaaaa 8760

ggatgtcgca aacgctgttt gctcctctac aaaacagacc ttaaaaccct aaaggcttaa 8820

gtagcaccct cgcaagctcg ggcaaatcgc tgaatattcc ttttgtctcc gaccatcagg 8880

cacctgagtc gctgtctttt tcgtgacatt cagttcgctg cgctcacggc tctggcagtg 8940

aatgggggta aatggcacta caggcgcctt ttatggattc atgcaaggaa actacccata 9000

atacaagaaa agcccgtcac gggcttctca gggcgtttta tggcgggtct gctatgtggt 9060

gctatctgac tttttgctgt tcagcagttc ctgccctctg attttccagt ctgaccactt 9120

cggattatcc cgtgacaggt cattcagact ggctaatgca cccagtaagg cagcggtatc 9180

atcaacaggc tta 9193

## Description

### [Technical Field]

The present disclosure relates to a microorganism having producing ability of quinolinic acid and a method for producing quinolinic acid using the microorganism.

### [Background Art]

Quinolinic acid is known as 2,3-pyridinedicarboxylic acid and is used as a precursor of chemical compounds used in a wide variety of fields such as medicine, agricultural chemicals, and dyeing materials.

The quinolinic acid can be prepared through chemical or biological synthesis methods. Since chemical synthesis methods use non-renewable materials derived from petroleum as raw materials, it has problems that are greatly affected by environmental problems and oil prices or petroleum extraction costs.

As a representative example of biological synthesis methods, there is a method for producing quinolinic acid in which genes encoding L-aspartate oxidase (NadB) and quinolinate synthase (NadA) are cloned into plasmids each having different copy numbers, and after enhancing the expression of the two enzymes in *Escherichia coli* in which the activity of quinolinate phosphoribosyltransferase is removed, quinolinic acid is produced from the strain (Eur. J. Biochem. 175, 221-228 (1988), DE3826041). However, the concentration of quinolinic acid in this case was 500 mg/L or less, which was a very low level.

The first cause for the production of quinolinic acid at such a low concentration is the inhibition of transcriptional stage expression regulation by NadR, which is an NAD-related transcriptional stage inhibition factor of *nadB* encoding L-aspartate oxidase and *nadA* gene encoding quinolinate synthase. The second cause is feedback inhibition of NadB protein, which is an L-aspartate oxidase, by NAD, and the third cause is determined to be that *Escherichia coli* used by itself has a weak biosynthetic pathway from carbon sources to L-aspartate.

### [Disclosure]

### [Technical Problem]

As a method for solving the first cause of the problem, the present inventors discovered a highly active exogenous quinolinate synthase and have made extensive efforts to increase the production amount of quinolinic acid using the same. As a result, when the activity of quinolinate synthase derived from *Klebsiella pneumoniae* was introduced into a microorganism having producing ability of quinolinic acid, it was found that the producing ability of quinolinic acid was more excellent compared to when quinolinate synthase derived from *Escherichia coli* was used, thereby completing the present disclosure.

### [Technical Solution]

An object of the present disclosure is to provide a microorganism having quinolinic acid productivity, which is transformed to have the activity of quinolinate synthase derived from *Klebsiella pneumoniae.*

Another object of the present disclosure is to provide a method for producing quinolinic acid using the microorganism having the producing ability of quinolinic acid.

### [Advantageous Effects]

The microorganism having the producing ability of quinolinic acid of the present disclosure can be valuably used for effective production of quinolinic acid.

### [Best Mode]

A specific aspect of the present disclosure is a microorganism having producing ability of quinolinic acid, which is transformed to have the activity of quinolinate synthase derived from *Klebsiella pneumoniae.*

As used herein, the term "quinolinate synthase" refers to an enzyme having an activity of synthesizing quinolinic acid from iminosuccinic acid. The EC number of the quinolinate synthase is 2.5.1.72, and it is also named as NadA. The activity of the quinolinate synthase is as follows.

### [Activity of quinolinate synthase]

α-iminosuccinate + dihydroxyacetone phosphate <=> quinolinic acid + phosphate + 2 H₂O.

The quinolinate synthase which is used in the present disclosure is quinolinate synthase derived from *Klebsiella,* and specifically, may be quinolinate synthase derived from *Klebsiella pneumoniae.* In the present disclosure, the quinolinate synthase derived from *Klebsiella pneumoniae* is also named as NadA(KP).

The sequence of the quinolinate synthase derived from *Klebsiella pneumoniae* can be easily obtained from databases known in the art such as the National Center for Biotechnology Information (NCBI) and DNA Data Bank of Japan (DDBJ), and examples include, but are not limited to, the sequence of a gene having the NCBI GenBank registration number of 339761016.

The quinolinate synthase derived from *Klebsiella pneumoniae* may be a protein having an amino acid sequence of SEQ ID NO: 1. Furthermore, as a protein having a homology of 80% or more, specifically 90% or more, more specifically 95% or more, and even more specifically 99% or more, to SEQ ID NO: 1, if it is an amino acid sequence having a biological activity substantially identical or corresponding to quinolinate synthase derived from *Klebsiella pneumoniae,* it is obvious that cases where some of the sequence has deletion, modification, substitution, or addition of amino acid sequences are included within the scope of the present disclosure.

In addition, a polynucleotide encoding the quinolinate synthase derived from *Klebsiella pneumoniae* may have a nucleotide sequence encoding the amino acid sequence of SEQ ID NO: 1. The polynucleotide may have various modifications in a coding region within a range that does not change the amino acid sequence of a protein, due to degeneracy of a codon or considering codon preference in an organism in which the protein is to be expressed. The polynucleotide sequence may have, for example, a nucleotide sequence of SEQ ID NO: 2 and may have a homology of 80% or more, specifically 90% or more, more specifically 99% or more, but is not limited thereto.

As used herein, the term "homology" refers to the percentage of identity between two polynucleotides or polypeptide moieties. The homology between sequences from one moiety to another can be determined by known techniques. For example, homology can be determined by directly aligning the sequence information between two polynucleotide molecules or two polypeptide molecules using an easily available computer program that is capable of aligning sequence information. The computer program may be BLAST (NCBI), CLC Main Workbench (CLC bio), MegAlign TM (DNASTAR, Inc.), *etc.* Further, homology between polynucleotides can be determined by hybridization of the polynucleotides under conditions that result in a stable double strand between homologous regions, followed by degradation by a single-strand-specific nuclease to determine the size of degraded fragments.

Specifically, the microorganism having producing ability of quinolinic acid transformed to have activity of quinolinate synthase derived from *Klebsiella pneumoniae* may have enhanced activity of quinolinate synthase compared to its endogenous activity.

As used herein, the term "endogenous activity" refers to an active state of a protein of interest in its native state, *i.e.,* in its non-variable state. "Enhanced, compared to endogenous activity" refers to an increase in activity when compared to the activity of the protein in its native state, and is a concept that also includes introducing the activity into a microorganism having no activity of the protein.

Enhancement of such activity can be accomplished by a variety of methods well known in the art, and for example, a method of inserting a polynucleotide comprising a nucleotide sequence encoding the protein into a chromosome, a method of introducing a polynucleotide encoding the protein into a vector system and thereby introducing it into a microorganism, a method of introducing a promoter having enhanced expression ability upstream of a polynucleotide encoding the protein or introducing a protein in which modifications are applied to a promoter, a method of introducing a variant of a polynucleotide encoding the protein, *etc.* may be used. Further, when the microorganism has the activity of the protein, a method of modifying the expression regulation sequence of a gene encoding the protein or a method of introducing a modification into a gene on a chromosome encoding the protein in order to enhance the activity of the protein, *etc.* may be performed, but the method is not limited by the above examples. Further, methods for enhancing such activity can be equally referenced when enhancing the activity of other proteins in the present specification.

For example, as the promoter having enhanced expression ability, known promoters may be used, and for example, the cj1 promoter (Korean Registered Patent No. 0620092), lac promoter, trp promoter, trc promoter, tac promoter, lambda phage PR promoter, PL promoter, and tet promoter may be included.

As used herein, the term "vector" refers to a DNA product containing a nucleotide sequence of a polynucleotide encoding a target protein operably linked to a suitable regulatory sequence so as to be capable of expressing the target protein within an appropriate host. The regulatory sequence includes a promoter capable of initiating transcription, any operator sequence for regulating such transcription, a sequence encoding a suitable mRNA ribosome binding site, and a sequence regulating the termination of transcription and translation. A vector may be transformed into a suitable host, then may be replicated or function, independent of the host genome, and may be integrated into the genome itself.

The vector used in the present disclosure is not particularly limited as long as it is replicable in a host, and any vector known in the art can be used. Examples of conventionally used vectors include plasmids, cosmids, viruses, and bacteriophages in their native or recombinant state. For example, pWE15, M13, λMBL3, λMBL4, λIXII, λASHII, λAPII, λt10, λt11, Charon4A, and Charon21A vector, *etc.* may be used as a phage vector or cosmid vector, and PBR, pUC, pBluescriptII, pGEM, pTZ, pCL, pET vector, *etc.* may be used as a plasmid vector. Specifically, pACYC177, pACYC184, pCL, pECCG117, pUC19, pBR322, pMW118, PCC1BAC vector, *etc.* can be used. However, the vector is not limited thereto.

As used herein, the term "transformation" refers to a series of operations in which a vector containing a polynucleotide encoding a target protein is introduced into a host cell so that a protein encoded by the polynucleotide can be expressed in the host cell. The polynucleotide introduced into the host cell may be in any form as long as it can be introduced into the host cell and expressed. For example, the polynucleotide may be introduced into the host cell in the form of an expression cassette which is a structure comprising all elements required to be self-expressed (a promoter operably linked to the polynucleotide, a transcription termination signal, a ribosome binding site, a translation termination signal, *etc.*), and the expression cassette may be in the form of an expression vector capable of self-replication. Further, the polynucleotide may also be introduced into the host cell in its own form and thereby be operably linked to the sequence necessary for expression in the host cell.

In addition, as used above, the term "operably linked" means that a promoter sequence, which initiates and mediates transcription of a polynucleotide encoding a target protein of the present disclosure, and the gene sequence are functionally linked.

Enhancement of such activity of the protein may be such that the activity or concentration of the corresponding protein is generally increased by at least 1%, 10%, 25%, 50%, 75%, 100%, 150%, 200%, 300%, 400%, or 500%, up to 1,000% or 2,000%, based on the activity or concentration in the initial microorganism strain, but is not limited thereto.

In addition, the microorganism having producing ability of quinolinic acid may further have an enhanced activity of L-aspartate oxidase as compared to its endogenous activity.

As used herein, the term "L-aspartate oxidase" refers to an enzyme having an activity of oxidizing L-aspartate to iminosuccinate. The L-aspartate oxidase has an EC number of 1.4.3.16 and can be named as NadB. The activity of L-aspartate oxidase is as follows.

### [Activity of L-aspartate oxidase]

L-aspartate + fumarate <=> α-iminosuccinate + succinate + H⁺

Or

L-aspartate + oxygen <=> hydrogen peroxide + α-iminosuccinate + H⁺

In addition, the sequence of the L-aspartate oxidase can be easily obtained from the genome sequence of *Escherichia coli* disclosed in the reference (Mol. Syst. Biol. 2006; 2:2006.0007. Epub 2006 Feb 21) or from databases known in the art such as NCBI and DDBJ.

As an example, the L-aspartate oxidase may not only be a protein having an amino acid sequence of SEQ ID NO: 19, but also be a protein having a homology of 80% or more, specifically 90% or more, more specifically 95% or more, and even more specifically 99% or more, but is not limited thereto. Substantially, as long as it is an amino acid sequence having a biological activity identical or corresponding to the L-aspartate oxidase, it is obvious that cases where some of the sequence has deletion, modification, substitution, or addition of amino acid sequences are included within the scope of the present disclosure.

In addition, a polynucleotide encoding the L-aspartate oxidase may have a nucleotide sequence encoding an amino acid sequence of SEQ ID NO: 19. The polynucleotide may have various modifications in a coding region within a range that does not change the amino acid sequence of the protein, due to degeneracy of a codon or considering the codon which is preferred in an organism in which the protein is to be expressed. Further, the polynucleotide sequence may have a polynucleotide sequence of SEQ ID NO: 20, and may have a nucleotide sequence with a homology of 80% or more, specifically 90% or more, and more specifically 95% or more. However, the polynucleotide sequence is not limited thereto.

In addition, the microorganism of the genus *Escherichia* may further have a weakened activity of quinolinate phosphoribosyltransferase compared to its endogenous activity.

The quinolinate phosphoribosyltransferase refers to an enzyme having an activity of converting quinolinic acid into nicotinic acid mononucleotide. The EC number of the quinolinate phosphoribosyltransferase is 2.4.2.19 and is also named as NadC. The activity of quinolinate phosphoribosyltransferase is expressed as follows.

### [Activity of quinolinate phosphoribosyltransferase]

5-phospho-α-D-ribose 1-diphosphate + quinolinic acid + 2H⁺ <=> CO₂ + diphosphate + nicotinate mononucleotide

In addition, the sequence of the quinolinate phosphoribosyltransferase can be easily obtained from the genome sequence (GI: 89106990) of *Escherichia coli* disclosed in the reference (Mol. Syst. Biol. 2006; 2:2006.2007. Epub 2006 Feb 21) or databases known in the art such as NCBI and DDBJ, and as an example, it may not only have an amino acid sequence represented by SEQ ID NO: 3, but also have a protein having a homology of 80% or more, specifically 90% or more, more specifically 95% or more, and even more specifically 99% or more, but is not limited thereto.

In addition, the polynucleotide encoding the quinolinate phosphoribosyltransferase may have a nucleotide sequence encoding an amino acid sequence of SEQ ID NO: 3. The polynucleotide may have various modifications in a coding region within a range that does not change the amino acid sequence of the protein, due to degeneracy of a codon or considering the codon which is preferred in an organism in which the protein is to be expressed. Further, the polynucleotide sequence may have, for example, a polynucleotide sequence of SEQ ID NO: 4, and may have a nucleotide sequence with a homology of 80% or more, specifically 90% or more, more specifically 95% or more, but is not limited thereto.

By weakening the activity of NadC compared to its endogenous activity, the accumulation of quinolinic acid in cell can be increased.

The weakening of the activity of the protein compared to the endogenous activity is a concept including both cases where the activity is decreased or the activity is absent, compared to the activity of the protein of the original microorganism in its native state.

Such weakening of protein activity can be achieved by applying various methods well known in the field. As examples of the method, there are a method of deleting all or part of a gene on a chromosome encoding the protein including a case where the activity of the protein is removed; a method of replacing a gene encoding the protein on a chromosome with a gene mutated to reduce the activity of the enzyme; a method of introducing modifications into the expression regulation sequence of a gene on a chromosome encoding the protein; a method of replacing the expression regulation sequence of a gene encoding the protein with a sequence with weak activity or without activity (for example, a method of replacing the promoter of the gene with a promoter which is weaker than the endogenous promoter); a method of introducing an antisense oligonucleotide (for example, antisense RNA) which binds complementarily to a transcript of a gene on the chromosome and inhibits the translation of the mRNA to a protein; a method of artificially adding a sequence complementary to an SD sequence to the front of the SD sequence of the gene encoding the protein to form a secondary structure to make it impossible for ribosomes to attach; and a reverse transcription engineering (RTE) method in which a promoter is added to reverse the 3' end of the open reading frame (ORF) of the corresponding sequence, *etc.,* and it can also be achieved by a combination of these, but is not particularly limited by the above examples.

Specifically, the method of deleting all or part of a gene encoding a protein can be performed by replacing the polynucleotide encoding an endogenous target protein in a chromosome with a polypeptide, in which some nucleic acid sequences have been deleted, or a marker gene, through a vector for insertion into the chromosome in the microorganism. As an example of such a method, a method of deleting a gene by homologous recombination may be used, but is not limited thereto. Further, in the above, the term "part" varies depending on the type of polynucleotides and can be appropriately determined by those skilled in the art, and specifically, it may be 1 to 300, more specifically 1 to 100, and even more specifically 1 to 50, but is not limited thereto.

In addition, a method of modifying an expression regulation sequence can be performed by inducing modifications in the expression regulation sequence by deletion, insertion, non-conservative or conservative substitution, or a combination thereof, in the nucleic acid sequence to further weaken the activity of the expression regulation sequence, or by replacing with a nucleic acid sequence having much weaker activity. The expression regulation sequence may include a promoter, an operator sequence, a sequence encoding a ribosome binding site, and a sequence regulating the termination of transcription and translation, but is not limited thereto.

Further, a method of modifying a gene sequence on a chromosome can be performed by inducing modifications in the sequence by deletion, insertion, non-conservative or conservative substitution, or a combination thereof, to further weaken the activity of the protein, or by replacing with an improved gene sequence to have weaker activity or with an improved gene sequence without any activity, but is not limited thereto.

In addition, the microorganism of the genus *Escherichia* may additionally have an enhanced activity of phosphoenolpyruvate carboxylase (PPC) or L-aspartate transaminase compared to the endogenous activity.

The phosphoenolpyruvate carboxylase is an enzyme that mediates the reaction to produce oxaloacetic acid from phosphoenolpyruvate and CO₂. The EC number of the phosphoenolpyruvate carboxylase is 4.1.1.31 and is also named as PPC.

### [Activity of phosphoenolpyruvate carboxylase]

Phosphoenolpyruvate + CO₂ -> oxaloacetic acid + phosphate

In addition, L-aspartate transaminase has an activity of synthesizing L-aspartate from phosphoenolpyruvate. The EC number of the L-aspartate transaminase is 2.6.1.1, and it can also be named as AspC or L-aspartate aminotransferase.

### [Activity of L-aspartate transaminase]

Oxaloacetic acid + glutamic acid <=> L-aspartic acid + 2-ketoglutamic acid

The sequences of the genes *ppc* and *aspC* encoding the enzymes can be obtained from the genome sequence (gi: 89110074, GI: 89107778) disclosed in the reference (Mol. Syst. Biol. 2006;2:2006.0007. Epub 2006 Feb 21.) or from databases such as NCBI and DDBJ.

The PPC and AspC mediate the synthesis of L-aspartic acid, which is a precursor of quinolinic acid, from phosphoenolpyruvate, and when their activity is enhanced, the production of L-aspartic acid, which is a precursor of quinolinic acid in the cell, can be increased, and thereby the production of quinolinic acid can be increased.

As used herein, the term "microorganism having producing ability of quinolinic acid " refers to a microorganism capable of producing quinolinic acid from carbon sources in a medium. Further, the microorganism producing quinolinic acid may be a recombinant microorganism. Specifically, the microorganism producing quinolinic acid is not particularly limited in its type, as long as it can produce quinolinic acid, and it may be a microorganism belonging to the genus *Enterobacter,* the genus *Escherichia,* the genus *Erwinia,* the genus *Serratia,* the genus *Providencia,* the genus *Corynebacterium,* and the genus *Brevibacterium,* and specifically, may be a microorganism belonging to the genus *Escherichia,* more specifically, may be *Escherichia coli,* but is not limited thereto.

Another specific aspect of the present disclosure is a method for producing quinolinic acid using the microorganism having producing ability of quinolinic acid, and further having the activity of quinolinate synthase derivedfrom *Klebsiella pneumoniae.*

Specifically, the method may include (a) culturing the microorganism in a medium; and (b) recovering quinolinic acid from the cultured microorganism, medium, or both of step (a).

Culture of the microorganism may be performed according to a suitable medium and culture conditions known in the art. Such a culture process can be easily adjusted and used according to the microorganism selected by those skilled in the art. Methods of culturing include batch culture, continuous culture, and fed-batch culture, but are not limited thereto. Various methods of culturing microorganisms are disclosed, for example, in "Biochemical Engineering" by James M. Lee, Prentice-Hall International Editions, pp 138-176.

The medium used for the culture may be a medium suitably satisfying the requirements of a specific microorganism. Media for various microorganisms are disclosed in the reference ("Manual of Methods for General Bacteriology" by the American Society for Bacteriology, Washington D.C., USA, 1981). The media comprise various carbon sources, nitrogen sources, and trace element components.

Carbon sources that can be used in a medium for culturing the microorganism include carbohydrates such as glucose, sucrose, lactose, fructose, maltose, starch, and cellulose; lipids such as soybean oil, sunflower oil, castor oil, and coconut oil; fatty acids such as palmitic acid, stearic acid, and linoleic acid; glycerol; alcohols such as ethanol; and organic acids such as acetic acid, but are not limited thereto. The carbon source may be used alone or in combination.

Nitrogen sources that can be used in a medium for culturing the microorganism include organic nitrogen sources and elements such as peptone, yeast extract, gravy, malt extract, corn steep liquor (CSL), and soybean wheat; and inorganic nitrogen sources such as ammonium sulfate, ammonium chloride, ammonium phosphate, ammonium carbonate, and ammonium nitrate, but are not limited thereto. The nitrogen source may be used alone or in combination.

The medium for culturing the microorganism may include potassium dihydrogen phosphate, dipotassium hydrogen phosphate, and corresponding sodium-containing salts as a source of phosphorous. Further, it may include metal salts such as magnesium sulfate or iron sulfate. In addition, amino acids, vitamins, and suitable precursors, *etc.* may be included in the medium, but the medium is not limited thereto. The medium for culturing the microorganism or individual components may be added to a culture solution either batchwise or continuously.

In addition, during culture, compounds such as ammonium hydroxide, potassium hydroxide, ammonia, phosphoric acid, and sulfuric acid may be added to the microbial culture solution in an appropriate manner to adjust the pH of the culture solution. Further, bubble formation can be suppressed by using a defoaming agent such as fatty acid polyglycol ester during the culture. In order to maintain the aerobic condition of the culture solution, oxygen or an oxygen-containing gas (for example, air) may be injected into the culture solution. The temperature of the culture solution may conventionally be in a range of 20°C to 45°C, specifically 25°C to 40°C. The culture period may be continued until the desired amount of quinolinic acid is obtained, and may specifically be in a range of 10 hours to 160 hours.

The step of recovering quinolinic acid in the step (b) may be performed through various methods well known in the art and may include a purification step.

### [Detailed Description of the Invention]

Hereinafter, the present disclosure will be described in detail with reference to the following exemplary embodiments. However, these exemplary embodiments are for explaining the present disclosure in more detail, and the scope of the present disclosure is not intended to be limited by these exemplary embodiments.

### Example 1. Preparation of strain producing quinolinic acid

### 1-1. Preparation of strain in which quinolinate phosphoribosyltransferase is removed

The following experiment was conducted in order to delete the *nadC* gene encoding quinolinate phosphoribosyltransferase involved in the degradation pathway of quinolinic acid.

Through performing PCR using the chromosomal DNA of *E. coli* K12 W3110 as a template, the *nadC* gene of the quinolinic acid degradation pathway was obtained. The nucleotide sequence information (NCBI registration number "GI: 89106990", SEQ ID NO: 4) of the *nadC* gene was obtained from GenBank of the National Institutes of Health (NIH), and the amino acid sequence thereof is the same as SEQ ID NO: 3. Based on SEQ ID NO: 4, primers of SEQ ID NOS: 5 and 6, which amplify a downstream region of the *nadC* gene, primers of SEQ ID NOS: 7 and 8, which amplify upstream and downstream regions of *nadC* and *loxpCm,* and primers of SEQ ID NOS: 9 and 10, which amplify an upstream region, were synthesized.

PCR was performed using the chromosomal DNA of *E. coli* K12 W3110 as a template and using the oligonucleotides of SEQ ID NOS: 5 and 6, and 9 and 10 as primers, to amplify upstream and downstream regions of the *nadC* gene of 0.5 kb and 0.3 kb, respectively. Further, by using pLoxpCat2 vector, which is a plasmid vector containing *loxpCm* (Genbank Accession No. AJ401047) as a template, and by using the oligonucleotides of SEQ ID NOS: 7 and 8 as primers, PCR was performed to amplify the *loxpCm* gene having a homologous sequence of the *nadC* gene at both ends of 1.0 kb. PfuUltra™ DNA polymerase (Stratagene, USA) was used as a polymerase, and PCR was performed by repeating 30 times a cycle consisting of denaturation for 30 seconds at 96°C, annealing for 30 seconds at 53°C, and extension for 1 minute at 72°C.

Thereafter, PCR was performed using a *nadC*-upstream fragment, a *nadC*-downstream fragment, and a *loxpCm* fragment, which were obtained through the above PCR reaction, as templates, and the PCR condition was repeating 10 times a cycle consisting of denaturation for 60 seconds at 96°C, denaturation for 60 seconds at 50°C, and extension for 1 minute at 72°C, and then repeating the cycle 20 times after addition of the primers of SEQ ID NOS: 5 and 10. As a result, a *nadC*-deficient cassette containing 1.8 kb of *nadC* gene-upstream-*loxpCm-*downstream was obtained.

The prepared *nadC*-deficient cassette was transformed through electroporation on *E. coli* K12 W3110 containing pKD46 which is a lambda red recombinase expression vector, and it was spread on a Luria-Bertani (LB) plate medium (10 g/L of tryptone, 5 g/L of yeast extract, 10 g/L of NaCl, and 1.5% agar) containing chloramphenicol which is a selective marker, and after incubating overnight at 37°C, a strain showing resistance to chloramphenicol was selected.

By using the selected strain as a direct template, and by using primers of SEQ ID NOS: 6 and 9, PCR was performed under the same condition, and the deletion of the *nadC* gene was confirmed by confirming that the size of the gene was 1.6 kb in a case of a wild-type strain, and the size of the gene was 1.3 kb in a case of the *nadC*-removed strain, on a 1.0% agarose gel. It was named as W3110*-ΔnadC.*

### 1-2. Preparation of strain in which quinolinate synthase is removed

In order to confirm the activity of quinolinate synthase originated from *Klebsiella pneumoniae* of the present disclosure, the following experiment was performed to delete *nadA* encoding quinolinate synthase of the microorganism itself.

Through performing PCR using the chromosomal DNA of *E*. *coli* K12 W3110 as a template, the *nadA* gene of quinolinate synthase was obtained. The nucleotide sequence information (NCBI registration number "GI: 89107601", SEQ ID NO: 12) of the *nadA* gene was obtained from GenBank of the National Institutes of Health (NIH), and the amino acid sequence thereof is the same as SEQ ID NO: 11. Based on SEQ ID NO: 12, primers of SEQ ID NOS: 13 and 14, which amplify a downstream region of the *nadA* gene, primers of SEQ ID NOS: 15 and 16, which amplify upstream and downstream regions of *nadA* and *loxpCm,* and primers of SEQ ID NOS: 17 and 18, which amplify an upstream region, were synthesized.

PCR was performed using the chromosomal DNA *of E. coli* W3110 as a template and using primers of SEQ ID NOS: 13 and 14, and 17 and 18, to amplify upstream and downstream regions of the *nadA* gene of 0.5 kb and 0.5 kb, respectively. Further, by using pLoxpCat2 vector, which is a plasmid vector containing *loxpCm* (Genbank Accession No. AJ401047) as a template, and by using the oligonucleotides of SEQ ID NOS: 15 and 16 as primers, PCR was performed to amplify the *loxpCm* gene having a homologous sequence of the *nadA* gene at both ends of 1.0 kb. PfuUltra™ DNA polymerase (Stratagene, USA) was used as a polymerase, and PCR was performed by repeating 30 times a cycle consisting of denaturation for 30 seconds at 96°C, annealing for 30 seconds at 53°C, and extension for 1 minute at 72°C.

Thereafter, PCR was performed using a *nadA*-upstream fragment, a *nadA*-downstream fragment, and a *loxpCm* fragment, which were obtained through the above PCR reaction, as templates, and the PCR condition was repeating 10 times a cycle consisting of denaturation for 60 seconds at 96°C, denaturation for 60 seconds at 50°C, and extension for 1 minute at 72°C, and then repeating the cycle 20 times after addition of primers of SEQ ID NOS: 13 and 18. As a result, a *nadA*-deficient cassette containing 2.0 kb of *nadA* gene-upstream-*loxpCm-*downstream was obtained.

The prepared *nadA*-deficient cassette was transformed through electroporation on *E. coli* W3110-ΔnadC containing pKD46 which is a lambda red recombinase expression vector, and it was spread on a Luria-Bertani (LB) plate medium (10 g/L of tryptone, 5 g/L of yeast extract, 10 g/L of NaCl, and 1.5% agar) containing chloramphenicol which is a selective marker, and after incubating overnight at 37°C, a strain showing resistance to chloramphenicol was selected.

By using the selected strain as a direct template, and by using primers of SEQ ID NOS: 14 and 17, PCR was performed under the same condition, and the deletion of the *nadA* gene was confirmed by confirming that the size of the gene was 1.1 kb in a case of a wild-type strain, and the size of the gene was 1.3 kb in a case of the *nadA*-removed strain, on a 1.0% agarose gel. It was named as W3110*-ΔnadCΔnadA.*

### 1-3. Preparation of E. coli L-aspartate oxidase expression vector

The following experiment was performed in order to enhance the *nadB* gene encoding L-aspartate oxidase.

A wild-type *nadB* gene originated from *E. coli* was cloned to an expression vector. As a template therefor, the chromosome of *E. coli* K12 W3110 strain (ATCC No. 23257) was used. The gene sequence was based on the nucleotide sequence (NCBI registration number "GI: 89109380", SEQ ID NO: 20) of the gene from GenBank of the National Institutes of Health (NIH), and the amino acid sequence is the same as SEQ ID NO: 19. Primers of SEQ ID NOS: 21 and 22 having recognition sites of restriction enzymes *NdeI* and *BamHI,* which amplify the ORF region of *nadB* gene were synthesized.

PCR was performed using the chromosomal DNA of the *E. coli* K12 W3110 as a template and using the oligonucleotides of SEQ ID NOS: 21 and 22 as primers. PfuUltra™ DNA polymerase (Stratagene, USA) was used as a polymerase, and PCR was performed by repeating 30 times a cycle consisting of denaturation for 30 seconds at 96°C, annealing for 30 seconds at 50°C, and extension for 2 minutes at 72°C. Through performing PCR, an amplified gene of about 1.9 kb containing the *nadB* ORF gene and the recognition sites of restriction enzymes *NdeI* and *BamHI* was obtained.

The *nadB* gene obtained through the PCR was recovered through agarose gel elution, followed by treatments with restriction enzymes *NdeI* and *BamHI.* Thereafter, it was ligated to a pProLar vector (CloneTech, USA) which was treated with restriction enzymes *NdeI* and *BamHI,* and L-aspartate oxidase was expressed from the *nadB* gene linked to a pPro promoter. The vector prepared by the above method was named as pPro-nadB vector.

### 1-4. Preparation of vector in which quinolinate synthase is expressed

### (1) Preparation of pNadA-nadA vector

The *nadA* gene encoding a wild-type quinolinate synthase originated from *E. coli* was cloned to an expression vector. As a template therefor, the chromosome of *E. coli* K12 W3110 strain (ATCC NO. 23257) was used. The nucleotide sequence information of the *nadA* gene of SEQ ID NO: 12 (NCBI registration number "GI: 89107601") was obtained from GenBank of the National Institutes of Health (NIH). Further, based on this, primers of SEQ ID NOS: 25 and 26 having recognition sites of restriction enzymes *XbaI* and *BamHI,* which can amplify the ATG region of the *nadA* gene and ORF region containing TAA were synthesized.

PCR was performed using the chromosomal DNA of the *E. coli* W3110 as a template and using the oligonucleotides of SEQ ID NOS: 25 and 26 as primers. PfuUltra™ DNA polymerase (Stratagene, USA) was used as a polymerase, and PCR was performed by repeating 30 times a cycle consisting of denaturation for 30 seconds at 96°C, annealing for 30 seconds at 50°C, and extension for 2 minutes at 72°C. As a result, an amplified gene of about 1 kb containing the *nadA* gene and recognition sites of restriction enzymes *XbaI* and *BamHI* was obtained.

In addition, based on Mendoza-Vargas, et al., PLoS ONE, 4: e7526, a pNadA promoter was obtained through performing PCR using the chromosomal DNA *of E. coli* W3110 containing a pNadA promoter. In order to ligate the pNadA promoter and the *nadA gene* that is amplified above, primers of SEQ ID NOS: 23 and 24 having recognition sites of restriction enzymes *PstI* and *XbaI* were synthesized.

PCR was performed using the chromosomal DNA of the *E. coli* W3110 as a template and using the oligonucleotides of SEQ ID NOS: 23 and 24 as primers. PfuUltra™ DNA polymerase (Stratagene, USA) was used as a polymerase, and the PCR condition was repeating 30 times a cycle consisting of denaturation for 30 seconds at 96°C, annealing for 30 seconds at 50°C, and extension for 1 minute at 72°C. As a result, an amplified gene of about 0.3 kb containing a pNadA promoter and recognition sites of restriction enzymes *PstI* and *XbaI* was obtained.

The *nadA* gene obtained through the PCR was treated with restriction enzymes *XbaI* and *BamHI,* and amplified pNadA promoter fragments were treated with *PstI* and *XbaI.* The *nadA* and pNadA promoter fragments which were treated with the restriction enzymes were cloned into a pCL1920 vector through ligation to prepare a pNadA-nadA recombinant vector. This is the same as shown in SEQ ID NO: 27.

### (2) Preparation of pNadA-nadA (KP) vector

In order to prepare quinolinate synthase originated from *Klebsiella pneumoniae, nadA(KP)* gene encoding *Klebsiella* quinolinate synthase was cloned into an expression vector. For this, the chromosomal DNA of a *Klebsiella* strain was used as a template. For the strain, ATCC No. 25955 was purchased and used. The gene sequence used was SEQ ID NO: 2 of the nucleotide sequence of the gene of GenBank of the National Institutes of Health (NIH), and the amino acid sequence thereof was the same as SEQ ID NO: 1. For gene cloning, primers of SEQ ID NOS: 28 and 29 having recognition sites of restriction enzymes *XbaI* and *BamHI* capable of amplifying the *nadA(KP)* gene region were synthesized.

PCR was performed using the chromosomal DNAof*Klebsiella* as a template and using the oligonucleotides of SEQ ID NOS: 28 and 29 as primers. PfuUltra™ DNA polymerase (Stratagene, USA) was used as a polymerase, and PCR was performed by repeating 30 times a cycle consisting of denaturation for 30 seconds at 96°C, annealing for 30 seconds at 50°C, and extension for 2 minutes at 72°C. As a result, an amplified gene of about 1 kb containing the *nadA(KP)* gene and recognition sites of restriction enzymes *XbaI* and *BamHI* was obtained.

The *nadA(KP)* gene obtained through the PCR was treated with restriction enzymes *XbaI* and *BamHI,* and a pNadA-nadA(KP) recombinant vector was prepared by ligating *nadA(KP)* fragments treated with restriction enzymes to a pCL1920 vector containing a pNadA promoter. This nucleotide sequence is the same as shown in SEQ ID NO: 30.

### 1-5. Preparation of plasmid for expression of aspartate oxidase and quinolinate synthase

### (1) Preparation of pPro-nadB-pCJ1-nadA vector

In order to produce quinolinic acid, enhancement of two enzymes, that is, aspartate oxidase and quinolinate synthase, is necessary. Therefore, a plasmid was prepared in which the *nadB* and *nadA* genes encoding these two enzymes could be expressed together. First, the *nadA* gene encoding quinolinate synthase was obtained through performing PCR using the chromosomal DNA *of E. coli* W3110 as a template. The nucleotide sequence information of the *nadA* gene (NCBI registration number "GI: 89107601") was used from GenBank of the National Institutes of Health (NIH), and it is the same as SEQ ID NO: 12. Based on this, primers of SEQ ID NOS: 31 and 32 having recognition sites of restriction enzymes *ApaI* and *NotI,* which can amplify the ATG region of the *nadA* gene and ORF region containing TAA were synthesized.

PCR was performed using the chromosomal DNA of *E. coli* W3110 as a template and using the oligonucleotides of SEQ ID NOS: 31 and 32 as primers. PfuUltra™ DNA polymerase (Stratagene, USA) was used as a polymerase, and PCR was performed by repeating 30 times a cycle consisting of denaturation for 30 seconds at 96°C, annealing for 30 seconds at 50°C, and extension for 2 minutes at 72°C. As a result, an amplified gene of about 1.0 kb containing the *nadA* gene and recognition sites of restriction enzymes *ApaI* and *NotI* was obtained.

Based on the Korean Registered Patent No. 0620092, a pCJ1 promoter was obtained through performing PCR using the plasmid DNA containing a pCJ1 promoter as a template. In order to ligate the *nadA* gene, which is amplified above, with a pCJ1 promoter, primers of SEQ ID NO: 33 and 34 having recognition sites of restriction enzymes *BamHI* and *ApaI* were synthesized.

PCR was performed using the chromosomal DNA of *E. coli* W3110 as a template and using the oligonucleotides of SEQ ID NOS: 33 and 34 as primers. PfuUltra™ DNA polymerase (Stratagene, USA) was used as a polymerase, and the PCR condition was repeating 30 times a cycle consisting of denaturation for 30 seconds at 96°C, annealing for 30 seconds at 50°C, and extension for 1 minute at 72°C. As a result, an amplified gene of about 0.3 kb containing the pCJ1 promoter and recognition sites of restriction enzymes *BamHI* and *ApaI* was obtained.

The *nadA* gene obtained through performing the PCR was treated with restriction enzymes *ApaI* and *NotI,* and the amplified pCJ1 promoter fragments were treated with *ApaI* and *BamHI.* The *nadA* and pCJ1 promoter fragments which were treated with the restriction enzymes were cloned into the pPro-nadB vector obtained in Example 1-3, which was treated with *NotI* and *BamHI,* through ligation, and finally a pPro-nadB_pCJ1-nadA recombinant vector of 5.9 kb was prepared, in which the *nadB* gene, whose expression is regulated by the pPro promoter as a constitutive promoter, and the *nadA* gene, whose expression is regulated by the pCJ1 gene promoter, were cloned.

### (2) Preparation of pPro-nadB-pCJl-nadA(KP) vector

In order to produce quinolinic acid, enhancement of two enzymes, that is, aspartate oxidase and quinolinate synthase, is necessary. For the preparation of a plasmid in which *nadB* and *nadA(KP)* encoding these two enzymes could be expressed together, the *nadA(KP)* gene encoding quinolinate synthase was obtained through performing PCR using the chromosomal DNA of a *Klebsiella* strain as a template. The sequence used was the nucleotide sequence (NCBI registration number "GI: 339761016") of the gene of GenBank of the National Institutes of Health (NIH), and it is the same as SEQ ID NO: 2. Based on this, primers of SEQ ID NOS: 35 and 36 having recognition sites of restriction enzymes *ApaI* and *NotI,* which can amplify ATG region of *nadA(KP)* gene and ORF region containing TAA were synthesized.

PCR was performed using the chromosomal DNA of the *Klebsiella* strain as a template and using the oligonucleotides of SEQ ID NOS: 35 and 36 as primers. PfuUltra™ DNA polymerase (Stratagene, USA) was used as a polymerase, and PCR was performed by repeating 30 times a cycle consisting of denaturation for 30 seconds at 96°C, annealing for 30 seconds at 50°C, and extension for 2 minutes at 72°C. As a result, an amplified gene of about 1.0 kb containing the *nadA* promoter and recognition sites of restriction enzymes *ApaI* and *NotI* was obtained.

The *nadA(KP)* gene obtained through the PCR was treated with restriction enzymes *ApaI* and *NotI,* and was cloned into the pPro-nadB-pCJ1-nadA vector obtained in Example 1-5(1) through ligation, and finally a pPro-nadB_pCJ1-nadA(KP) recombinant vector of 5.9 kb was prepared, in which the *nadB* gene, whose expression is regulated by the pPro promoter as a constitutive promoter, and the *nadA(KP)* gene, whose expression is regulated by the pCJ1 gene promoter, were cloned.

### 1-6. Preparation of plasmid for expressions of phosphoenolpyruvate carboxylase and L-aspartate transaminase

Through performing PCR using the chromosomal DNA of *E*. *coli* W3110 as a template, the *ppc* gene encoding phosphoenolpyruvate carboxylase was obtained. The nucleotide sequence of the *ppc* gene (NCBI registration number "GI: 89110074") of SEQ ID NO: 37 was obtained from GenBank of the National Institutes of Health (NIH), and based on this, primers of SEQ ID NOS: 38 and 39 having recognition sites of restriction enzymes *HindIII* and *BamHI,* which can amplify a region containing the promoter of the *ppc* gene to the terminator, were synthesized.

PCR was performed using the chromosomal DNA of *E*. *coli* W3110 strain as a template and using the oligonucleotides of SEQ ID NOS: 38 and 39 as primers. PfuUltra™ DNA polymerase (Stratagene, USA) was used as a polymerase, and PCR was performed by repeating 30 times a cycle consisting of denaturation for 30 seconds at 96°C, annealing for 30 seconds at 50°C, and extension for 4 minutes at 72°C. As a result, an amplified gene of about 3.1 kb containing the *ppc* gene and recognition sites of restriction enzymes *HindIII* and *BamHI* was obtained. The *ppc* gene which was obtained through the PCR was treated with restriction enzymes *HindIII* and *BamHI,* and was cloned into a PCL1920(AB236930) vector, which was treated with restriction enzymes *HindIII* and *BamHI,* through ligation, and finally, a pCP recombinant vector was prepared, in which the *ppc* gene was cloned.

In order to clone the *aspC* gene on the pCP recombinant vector in which the *ppc* gene was cloned, the *aspC* gene encoding L-aspartate transaminase was obtained through performing PCR using the chromosomal DNA of *E. coli* W3110 as a template. The nucleotide sequence of the *aspC* gene (NCBI registration number "GI 89107778") of SEQ ID NO: 40 was obtained from GenBank of the National Institutes of Health (NIH), and based on this, primers of SEQ ID NOS: 41 and 42 having recognition sites of restriction enzymes *BamHI* and *KpnI,* which can amplify a region containing the promoter of the *aspC* gene to the terminator, were synthesized.

PCR was performed using the chromosomal DNA of *E. coli* W3110 as a template and using the oligonucleotides of SEQ ID NOS: 41 and 42 as primers. PfuUltra™ DNA polymerase (Stratagene, USA) was used as a polymerase, and PCR was performed by repeating 30 times a cycle consisting of denaturation for 30 seconds at 96°C, annealing for 30 seconds at 50°C, and extension for 2 minutes at 72°C. As a result, an amplified gene of about 1.5 kb containing the *aspC* gene and recognition sites of restriction enzymes *BamHI* and *KpnI* was obtained.

The *aspC* gene obtained through the PCR was treated with restriction enzymes *BamHI* and *KpnI,* and was cloned through ligation to the pCP vector which was treated with restriction enzymes *BamHI* and *KpnI,* and finally, a pCPA recombinant vector was prepared, in which the *aspC* gene and *ppc* gene were simultaneously cloned. The prepared pCPA vector has a sequence of SEQ ID NO: 43.

### Example 2. Evaluation of productivity of quinolinic acid-producing strain

In order to evaluate the ability of the *nadA(KP)* gene-enhanced strain to produce quinolinic acid, pNadA-nadA and pNadA-nadA(KP) vectors were introduced into the W3110-*ΔnadC* strain, and W3110ΔnadC/pNadA-nadA and W3110ΔnadC/pNadA-nadA(KP) strains were named as CV01-0812 and CV01-0813, respectively. In addition, for the enhancement of *nadB,* a pPro-nadB vector was introduced into CV01-0812 and CV01-0813, and they were named as CV01-0814 and CV01-0815, respectively.

The introduction method of vectors was transformation using the CaCl₂ method, and in an incubator at 37°C, CV01-0812 and CV01-0813 were spread on an LB-Sp (10 g/L of yeast extract, 5 g/L of NaCl, 10 g/L of Tryptone, 1.5% agar, 50 µg/L of spectinomycin) plate medium and cultured overnight, and CV01-0814 and CV01-0815 were spread on an LB-sp, Km (10 g/L of yeast extract, 5 g/L of NaCl, 10 g/L of Tryptone, 1.5% agar, 50 µg/L of kanamycin, 50 µg/L of spectinomycin) plate medium and cultured overnight. Thereafter, an obtained single colony having antibiotic resistance was inoculated by a platinum loop into 25 mL of a quinolinic acid titer medium and cultured for 24 hours to 72 hours at 250 rpm at 33°C. Table 1 below represents the composition of the medium for producing quinolinic acid.

**[Table 1]**

| Composition of quinolinic acid titer medium | |
|---|---|
| Composition | Concentration (per liter) |
| Glucose | 70 g |
| Ammonium sulfate | 17 g |
| KH₂PO₄ | 1.0 g |
| MgSO₄ ·7 H₂O | 0.5 g |
| FeSO₄ · 7 H₂O | 5 mg |
| MnSO₄ · 8 H₂O | 5 mg |
| ZnSO₄ | 5 mg |
| Calcium carbonate | 30 g |
| Yeast extract | 2 g |
| Methionine | 0.15 g |

Quinolinic acid in the culture solution was analyzed by HPLC, and the results are shown in Table 2 below. As can be confirmed in Table 2, the necessity of simultaneous enhancement of *nadB* and *nadA* in the production of quinolinic acid was confirmed, and it was confirmed that the *Klebsiella-based nadA(KP)-enhanced* strain showed approximately a 10% increase in the production of quinolinic acid compared to the wild-type *nadA*-enhanced strain.

**[Table 2]**

| Strain | Quinolinic acid (g/L) |
|---|---|
| CV01-0812 | 0.1 |
| CV01-0813 | 0.1 |
| CV01-0814 | 5.6 |
| CV01-0815 | 6.1 |

To evaluate the activity of single-species quinolinate synthase, pNadA-nadA and pNadA-nadA(KP) vectors were transformed into W3110-*ΔnadCΔnadA* strains containing a pPro-nadB vector, respectively, using the CaCl₂ method, and W3110*ΔnadCΔnadA* / pNadA-nadA, pPro-nadB and W3110*ΔnadCΔnadA* / pNadA-nadA(KP), pPro-nadB strains were named as CV01-0816 and CV01-0817, respectively. Among these, the CV01-0817 strain was deposited under the Budapest Treaty on November 27, 2014, with the Korean Culture Center of Microorganisms (KCCM) and was granted an accession number of KCCM11612P.

Using the above two strains, evaluation of a quinolinic acid titer medium was conducted as follows.

The transformed CV01-0816 and CV01-0817 strains were spread on an LB-Km, Sp (10 g/L of yeast extract, 5 g/L of NaCl, 10 g/L of Tryptone, 1.5% agar, 50 µg/L of kanamycin, 50 µg/L of spectinomycin) plate medium and cultured overnight in an incubator at 37°C. Thereafter, an obtained single colony having kanamycin and spectinomycin resistances was inoculated by a platinum loop into 25 mL of a quinolinic acid titer medium and cultured for 24 hours to 72 hours at 250 rpm at 33°C.

Quinolinic acid in the culture solutions was analyzed by HPLC, and the results are shown in Table 3. As can be confirmed in Table 3, when *Klebsiella*-based *nadA(KP)* was enhanced, the production of quinolinic acid increased by more than 9% compared to when *nadA* was enhanced.

**[Table 3]**

| Strain | Quinolinic acid (g/L) |
|---|---|
| CV01-0816 | 6.1 |
| CV01-0817 | 6.5 |

Additionally, in order to evaluate the producing ability of quinolinic acid when enhancing the biosynthetic pathway, a pCPA vector was introduced into W3110-ΔnadC and W3110-*ΔnadCΔnadA* strains, and further, pPro-nadB-pCJ1-nadA and pPro-nadB-pCJ1-nadA(KP) vectors, in which *nadB* and *nadA* were simultaneously enhanced, were introduced, respectively. W3110-ΔnadC/pCPA, pPro-nadB-pCJ1-nadA, W3110-ΔnadC/pCPA, pPro-nadB-pCJ1-nadA(KP), W3110-ΔnadCΔnadA/pCPA, pPro-nadB-pCJ1-nadA, and W3110-*Δ nadCΔnadA*/pCPA, and pPro-nadB-pCJ1-nadA(KP) strains were named as CV01-0818, CV01-0819, CV01-0820, and CV01-0821, respectively.

The introduction method was transformation using the CaCl₂ method, and these strains were spread on an LB-Km, Sp (10 g/L of yeast extract, 5 g/L of NaCl, 10 g/L of tryptone, 50 µg/L of kanamycin, 50 µg/L of spectinomycin) plate medium and cultured overnight in an incubator at 37°C. Thereafter, an obtained single colony having kanamycin and spectinomycin resistances was inoculated by a platinum loop into 25 mL of a quinolinic acid titer medium and cultured for 24 hours to 72 hours at 250 rpm at 33°C.

Quinolinic acid in the culture solutions was analyzed by HPLC, and the results are shown in Table 4 below. As can be seen in Table 4 below, it was confirmed that when *Klebsiella-based nadA(KP)* was enhanced, the production of quinolinic acid was increased by more than 10% compared to when *nadA* was enhanced.

**[Table 4]**

| Strain | Quinolinic acid (g/L) |
|---|---|
| CV01-0818 | 7.1 |
| CV01-0819 | 7.8 |
| CV01-0820 | 7.3 |
| CV01-0821 | 8.1 |

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

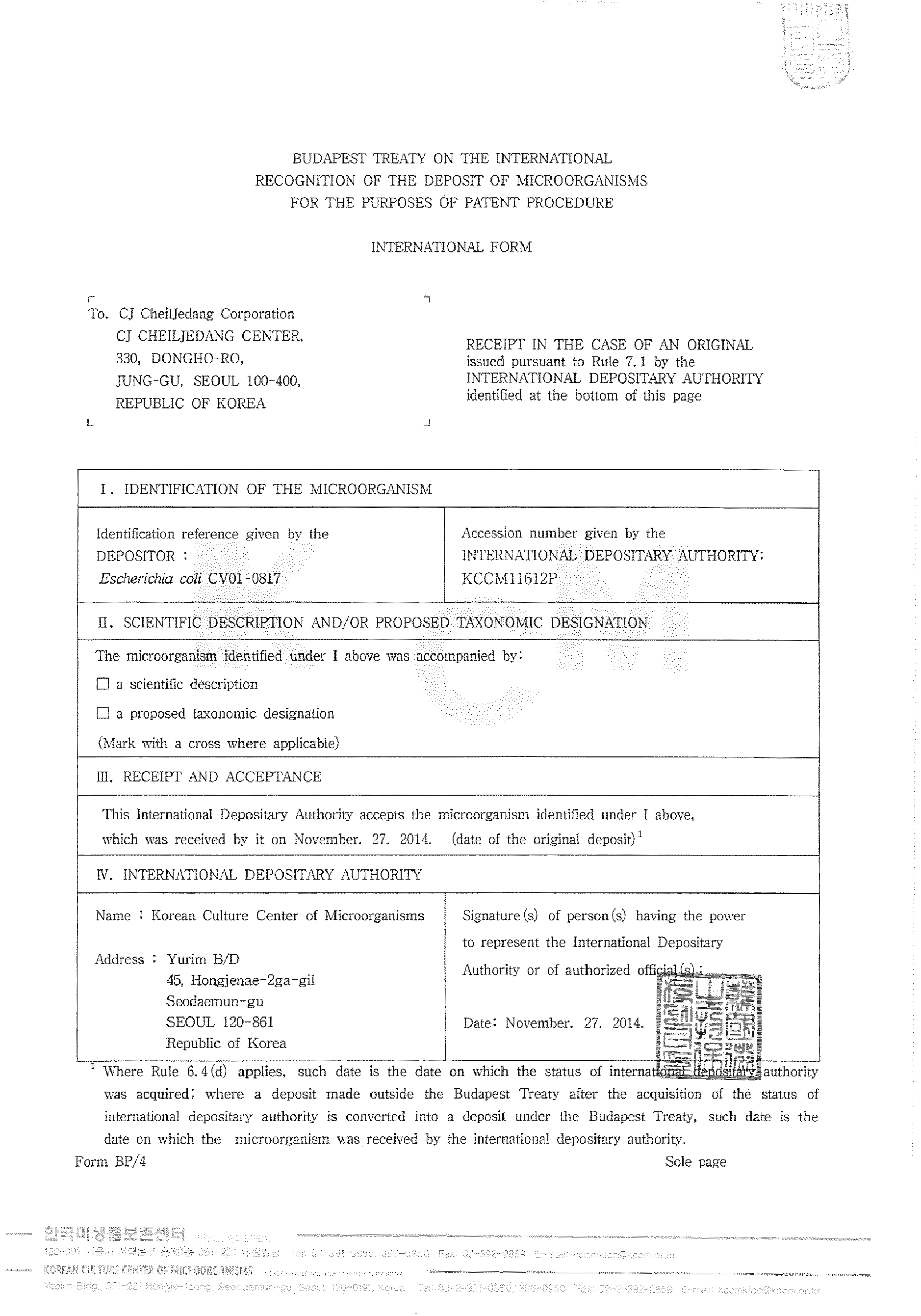

## Claims

1. A microorganism of the genus *Escherichia* having producing ability of quinolinic acid, which is transformed to have an activity of quinolinate synthase derived from *Klebsiella pneumoniae.*

2. The microorganism of the genus *Escherichia* according to claim 1, wherein the quinolinate synthase derived from *Klebsiella pneumoniae* has an amino acid sequence of SEQ ID NO: 1.

3. The microorganism of the genus *Escherichia* according to claim 1, wherein a polynucleotide encoding the quinolinate synthase derived from *Klebsiella pneumonia* has a nucleotide sequence of SEQ ID NO: 2.

4. The microorganism of the genus *Escherichia* according to claim 1, wherein the microorganism of the genus *Escherichia* further has an enhanced activity of L-aspartate oxidase compared to its endogenous activity.

5. The microorganism of the genus *Escherichia* according to claim 1, wherein the microorganism of the genus *Escherichia* further has a weakened activity of quinolinate phosphoribosyltransferase compared to its endogenous activity.

6. The microorganism of the genus *Escherichia* according to claim 1, wherein the microorganism of the genus *Escherichia* is *Escherichia coli.*

7. A method for producing quinolinic acid, comprising:
(a) culturing the microorganism of the genus *Escherichia* according to any one of claims 1 to 6 in a medium; and
(b) recovering quinolinic acid from the cultured microorganism, the medium, or both of step (a).
